# EUROPÄISCHE PATENTSCHRIFT

(11) **EP 1 890 682 B1**
(45) Veröffentlichungstag und Bekanntmachung des Hinweises auf die Patenterteilung: **03.05.2017**
(21) Anmeldenummer: 06723193.6
(22) Anmeldetag: 03.03.2006
(51) Int. Cl.: A61K 9/52

(54) **VERWENDUNG VOM POLYMERMISCHUNGEN ZUR HERSTELLUNG VON ÜBERZOGENEN ARZNEIFORMEN SOWIE ARZNEIFORM MIT**
USE OF POLYMER MIXTURES FOR THE PRODUCTION OF COATED PHARMACEUTICAL FORMULATIONS AND PHARMACEUTICAL FORMULATION WITH MIXED POLYMERIC COATING
UTILISATION DE MÉLANGES POLYMÈRES POUR LA PRODUCTION DE FORMES GALÉNIQUES ENROBÉES ET FORME GALÉNIQUE AINSI OBTENUE

(30) Priorität: 25.05.2005 DE 102005024614
(43) Veröffentlichungstag der Anmeldung: 27.02.2008
(73) Patentinhaber: Evonik Röhm GmbH, 64293 Darmstadt (DE)
(72) Erfinder: MEIER, Christian, 64295 Darmstadt (DE); KNUPPEN, Karin, 61449 Steinbach (DE); PETEREIT, Hans-Ulrich, 64291 Darmstadt (DE)
(86) Internationale Anmeldenummer: PCT/EP2006/001949
(87) Internationale Veröffentlichungsnummer: WO 2006/125483

(56) Entgegenhaltungen:
- EP-A- 0 347 024
- EP-A- 0 888 772
- WO-A-01/15683
- WO-A-01/68058
- WO-A-03/007917
- WO-A-2004/041255
- WO-A-2004/062577
- WO-A-2005/041934
- DE-A1- 3 943 242

## Beschreibung

Die Erfindung betrifft die Verwendung von Polymermischungen zur Herstellung von überzogenen Arzneiformen sowie eine Arzneiform mit polymerem Mischüberzug.

### Stand der Technik

Die Verwendung von sogenannten neutralen Methacrylat-Copolymeren, das heißt Methacrylat-Copolymeren, die zum überwiegenden Teil aus (mindestens 95%) (Meth)acrylat-Monomeren mit neutralen Resten, wie Methylmethacrylat oder Ethylacrylat, bestehen, als Überzugs- und Bindemittel für Arzneiformen mit verzögerter Wirkstofffreisetzung ist seit langem bekannt. Verwendungen in Mischungen mit anionischen Dispersionen sind beschrieben z. B. in EP-A 152 038, EP-A 208 213 oder EP-A 617 972. WO 01/68767 beschreibt die Herstellung von Dispersionen, enthaltend neutrale Methylacrylat-Copolymere, unter Verwendung von 1 - 10 Gew.-% eines nichtionischen Emulgators mit einem HLB-Wert von 15, 2 bis 17,3. Diese Maßnahmen erlaubt es unter Beibehaltung der Stabilität der Dispersion und ihrer Teilchengrößenverteilung, daraus Arzneimittelformulierungen herzustellen, bei denen eine Phasenseparation unter Ausbildung von Kristallstrukturen durch den Emulgator unterbleibt.

EP 0 152 038 A2 beschreibt überzogene Arzneiformen mit Mischüberzügen aus wasserlöslichen carboxylgruppenhaltigen Polymeren und wasserunlöslichen, filmbildenden Polymeren. Die Polymere können in Verhältnissen von 60 : 40 bis 5 : 95 vorliegen. Beispielsweise werden Mischüberzüge aus Polymeren beschrieben, die zu einem aus gleichen Teilen Ethylacrylat und Methacrylsäure und zu anderen aus Polymeren bestehen können, die sich aus Ethylacrylat und Methylmethacrylat im Verhältnis von 2 zu 1 zusammensetzen.

EP 0 208 213 A1 ist nahe inhaltsgleich mit EP 0 152 038 A2, offenbart jedoch zusätzlich den Effekt hoher Dehnbarkeit und Elastizität entsprechender MischÜberzüge.

EP 0 704 208 A2 beschreibt Überzugs- und Bindemittel für darmsaftlösliche Arzneiumhüllungen. Es handelt sich dabei um Copolymerisate aus 10 bis 25 Gew.-% Methacrylsäure, 40 bis 70 Gew.-% Methylacrylat und 20 bis 40 Gew-% Methylmethacrylat. Die Beschreibung erwähnt neben einschichtigen Überzügen auch mehrlagige Überzugssysteme. Diese können aus einem Kern, der z. B. einen basischen oder einen wasserempfindlichen Wirkstoff enthält, bestehen, weisen eine Isolierschicht aus einem anderen Überzugsmaterial, wie Celluloseether, Celluloseester oder einem kationischen Polymethacrylat z. B. von Typ EUDRAGIT®, u. a. auch EUDRAGIT® RS und RL, auf und werden dann zusätzlich mit der oben genannten darmsaftlöslichen Umhüllung versehen.

WO 03/072087 beschreibt ein Verfahren zur Herstellung einer Arzneiform, bei dem ein Copolymer einsetzt wird, welches sich aus
20 bis 34 Gew.-% Methacrylsäure und/oder Acrylsäure,
20 bis 69 Gew.-% Methylacrylat und
0 bis 40 Gew.-% Ethylacrylat und/oder gegebenenfalls
0 bis 10 Gew.-% weiteren vinylisch copolymerisierbarer Monomeren
zusammensetzt, mit der Maßgabe, daß die Glastemperatur des Copolymers nach ISO 11357-2, Punkt 3.3.3, höchstens 60 °C beträgt.

Zur Steuerung der Wirkstoffabgabe kann dabei es im Einzelfall vorteilhaft sein, dem Copolymer weitere Polymere zuzumischen. Der Anteil weiterer Polymere an der Mischung kann in weiten Bereichen variieren und liegt zwischen 1 und 99%, bevorzugt zwischen 10 und 90 Gew.-%, insbesondere bevorzugt zwischen 25 und 85 Gew.-% .- bezogen auf die Polymermischung beträgt.

Beispiele für solche weiteren Polymere sind: Polyvinylpyrolidone, Polyvinylalkohole, anionische (Meth)acrylat-Copolymere aus Methylmethacrylat und/oder Ethylacrylat und Methacrylsäure (EUDRAGIT^{®} L 100, EUDRAGIT^{®} S 100, EUDRAGIT^{®} L 100-55). Anionische (Meth)acrylat-Copolymere aus Methylmethacrylat, Methylacrylat und Methacrylsäure des Standes der Technik (s. z. B. EP-A 0 704 207 oder EP-A 0 704 208), Carboxymethylcellulose-Salze, Hydroxypropylcellulose (HPMC), neutrale (Meth)acrylat Copolymere aus Methylmethacrylat und Ethylacrylat (Trockensubstanz aus EUDRAGIT^{®} NE 30 D), Copolymere aus Methylmethacrylat und Butylmethacrylat (PLASTOID^{®} B) oder (Meth)acrylat Copolymere mit quaternären Ammoniumgruppen (EUDRAGIT^{®} RL bzw. EUDRAGIT^{®} RS).

WO 2004/096185 beschreibt ein Verfahren zur Herstellung einer überzogenen Arzneiform oder einer Arzneiform in Form einer wirkstoffhaltigen Matrix, indem man ein Copolymer, einen pharmazeutischen Wirkstoff, einen gegebenenfalls vorhandenen Kern und/oder pharmazeutisch übliche Zuschlagstoffe in an sich bekannter Weise durch Schmelzen, Spritzguß, Extrusion, Feuchtgranulieren, Gießen, Tauchen, Ausstreichen, Aufsprühen oder Verpressen zu einer überzogenen Arzneiform und/oder zu einer wirkstoffhaltigen Matrix verarbeitet, wobei man ein Copolymer einsetzt, welches sich aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und
gegebenenfalls
0 bis 10 Gew.-% weiteren vinylisch copolymerisierbarer Monomeren, wobei sich die Anteile der Monomeren zu 100 Gew.-% addieren,
zusammensetzt, mit der Maßgabe, dass die Glastemperatur des Copolymers 55 bis 70 °C beträgt.

Zur Steuerung der Wirkstoffabgabe kann dabei es im Einzelfall vorteilhaft sein, dem Copolymer weitere Polymere zuzumischen. Der Anteil weiterer Polymere an der Mischung kann in weiten Bereichen variieren und liegt zwischen 5 und 95, bevorzugt zwischen 10 und 90 Gew.-%, insbesondere bevorzugt zwischen 25 und 85 Gew.-%.

Beispiele für solche weiteren Polymere sind: Polyvinylpyrolidone, Polyvinylalkohole, anionische (Meth)acrylat-Copolymere aus Methylmethacrylat und/oder Ethylacrylat und Methacrylsäure (EUDRAGIT^{®} L 100, EUDRAGIT^{®} S 100, EUDRAGIT^{®} L 100-55). Anionische (Meth)acrylat-Copolymere aus Methylmethacrylat, Methylacrylat und Methacrylsäure des Standes der Technik (s. z. B. EP-A 0 704 207 oder EP-A 0 704 208), Carboxymethylcellulose-Salze, Hydroxypropylcellulose (HPMC), neutrale (Meth)acrylat Copolymere aus Methylmethacrylat und Ethylacrylat (Trockensubstanz aus EUDRAGIT^{®} NE 30 D), Copolymere aus Methylmethacrylat und Butylmethacrylat (PLASTOID^{®} B) oder (Meth)acrylat Copolymere mit quaternären Aminogruppen (EUDRAGIT^{®} RL bzw. EUDRAGIT^{®} RS).

WO 2004/041255 A1 beschreibt eine Darreichungsform zur Freisetzung flüssiger Formulierungen. WO 01115683 A1 beschreibt eine retardierte Darreichungsform, enthaltend Tramadolsaccharinat. WO 2004/062577 beschreibt Mischungen von zwei oder mehr enterischen Materialien zur kontrollierten Wirkstoffreiselzung über Membranen oder Matrices für systemische Therapeutika.

### Aufgabe und Lösung

EP 0 152 038 A2 geht von Arzneiformen mit Überzügen aus carboxylgruppenhaltigen Polymeren aus. Diese carboxylgruppenhaltigen Polymere, insbesondere Methacrylsäure-haltige (Meth)acrylatcopolymere sind resistent gegenüber Magensäften und zugleich aber darmsaftlöslich. Je nach Gehalt an Carboxylgruppen lösen sie sich bei einem spezifischen pH-Werten auf. Mit einem Polymeren aus gleichen Teilen Ethylacrylat und Methacrylsäure überzogene Arzneiformen setzen den Wirkstoff z. B. ab etwa pH 5,5 rasch frei. Gemäß der EP 0 152 038 A2 wird beim Zumischen von wasserunlöslichen, filmbildenden Polymeren der Effekt beobachtet, dass sich der Auslöse pH-Wert nach oben verschiebt, die Wirkstofffreisetzungscharakteristik bzw. deren zeitlicher Verlauf aber im wesentlichen unbeeinflusst bleibt. Der Effekt der Mischung kann als "pH-Shift" beschrieben werden. Will man den zeitlichen Verlauf der Wirkstofffreisetzungscharakteristik beeinflussen, so ist dies offenbar nur durch die Abänderungen der Monomerzusammensetzung des carboxylgruppenhaltigen Polymeren möglich. Ein Fachmann auf dem Gebiet der Galenik sieht sich dem Problem gegenüber, dass er nur über eine begrenzte Anzahl von Polymeren verfügt. Er müsste deshalb neue Polymere mit neuen Monomerzusammensetzungen entwickeln, um Varianten zu erhalten, mit denen sich bei gleichem Auflöse pH-Werte anderer zeitliche Verläufe der Wirkstofffreisetzungscharakteristik darstellen lassen.

Es sollte daher eine Lösung gefunden werden, die es ermöglicht auf einfache Weise den zeitlichen Verlauf der Wirkstofffreisetzungscharakteristik von anionischen bzw. carboxylgruppenhaltigen Polymeren zu verändern, ohne dabei deren Auflöse-pH-Wert zu beeinflussen.

### Die Aufgabe wird gelöst durch die

Verwendung einer Mischung aus 2 bis 60 Gew.-% eines oder mehreren Polymeren (I) mit 40 bis 98 Gew.-% eines oder mehreren Polymeren (II), wobei
das Polymer (I) ein (Meth)acrylat-Copolymer ist, enthaltend 90 bis 100 Gew.-% radikalisch polymerisierte Einheiten aus 66 bis 95 Gew.-% von C₁- bis C₄-Alkylestern der Acryl- oder der Methacrylsäure und 5 bis 34 Gew.-% Einheiten von (Meth)acrylat-Monomeren mit einer anionischen Gruppe, und zu 0 bis 10 Gew.-% aus weiteren vinylisch polymerisierbaren Monomeren, und
das Polymer (II) ein vom Polymer (I) verschiedenes Vinylpolymer oder ein Polysaccharid oder ein Derivat eines Polysaccharids ist, enthaltend 88 bis 100 % neutrale Monomereinheiten und bis zu 12 Gew.-% polymerisierte Monomereinheiten mit ionischen Resten,
wobei das Polymer (II) ein Copolymer aus Methylmethacrylat und Ethylacrylat,, ein Copolymer aus Methylmethacrylat, Ethylacrylat und Trimethylammoniumethylmethacrylat, Polyvinylacetat (PVAc), Hydroxyethylcellulose (HEC), Ethylcellulose (EC) oder eine Mischung der genannten Polymere ist,
zur Herstellung einer überzogenen Arzneiform, bestehend auseinem wirkstoffhaltigen Kern und einem polymeren Überzug aus der Mischung der Polymere (I) und (II), wobei der Polymermischung pharmazeutisch übliche Hilfsstoffe beigemengt sein können,
dadurch gekennzeichnet, dass
die Glastemperatur des Polymeren (I) nicht mehr als 70 °C beträgt und ein Wirkstofffreigabeprofil erhalten wird, bei welchem der Wirkstoff im Vergleich zu einer mit dem Polymer (I) allein überzogenen Arzneiform, beginnend beim gleichen pH-Wert jedoch verlangsamt freigesetzt wird.

Die in der EP 0 152 038 A2 beschriebenen (Meth)acrylatcopolymere, z. B. EUDRAGIT® L oder EUDRAGIT^{®} L100-55, weisen Glastemperaturen von über 100 °C auf. Polymere dieses Typs sind als Polymer (I) für die Zwecke der Erfindung nicht geeignet. Der Erfindung liegt die Erkenntnis zugrunde, dass sich der in EP 0 152 038 A2 für die dort beschriebenen Polymermischungen beschriebene "pH-Shift"-Effekt nicht bei einer Auswahl von anionischen bzw. carboxylgruppenhaltigen Polymeren einstellt, deren Glastemperatur nicht mehr als 70 °C beträgt. Bei diesen Polymeren wird durchaus überraschenderweise der aufgabengemäße Effekt der Veränderung des zeitlichen Verlaufs der Wirkstofffreisetzungscharakteristik ohne Veränderung des Auflöse-pH-Werts gefunden.

Ein Teil der umfassten Polymermischungen sind im Prinzip aus WO 03/072087 und WO 2004/096185 bekannt. Gemäß der breit angelegten Lehre der EP 0 152 038 A2 war davon auszugehen, dass die dort beschriebenen Mischungen zu einem erfindungsgemäß nicht erwünschten pH-shift-Effekt führen würden. Die Verwendung ausgewählter Mischungen aus WO 03/072087 und WO 2004/096185 zur Lösung der gestellten Aufgabe eröffnet somit neue Aspekte für die Galenik. Der Fachmann kann ausgehend vom einem darmsaftlöslichen Wirkstofffreisetzungscharakteristik von anionischen bzw. carboxylgruppenhaltigen Polymeren mit zugeordneten spezifischen Auflöse-pH-Werten den zeitlichen Verlauf der Wirkstofffreisetzungscharakteristik über das Mischungsverhältnis der Polymere einstellen. Dadurch können aufwändige Alternativentwicklungen, spezielle komplizierte Überzugs- Formulierungen oder die Entwicklung von Polymeren mit alternativen Monomerzusammensetzung, vermieden werden.

Copolymere aus 10 bis 30 Gew.-%, Methylmethacrylat, 50 bis 70 Gew.-% Methylacrylat und 5 bis 15 Gew.-% Methacrylsäure sind aus EP 0 704 208 A2 bekannt. Mischungen mit anderen Polymeren, die dem Typ hier beschriebenen Polymer (II) entsprechen, sind offenbar bisher nicht diskutiert worden. Auch hier wäre gemäß der breit angelegten Lehre der EP 0 152 038 A2 zu erwarten gewesen, dass solche Mischungen zu dem bekannten pH-shift-Effekt führen würden. Auch hier liegt der Erfindung die Erkenntnis zugrunde, dass sich der in EP 0 152 038 A2 für die dort beschriebenen Polymermischungen beschriebene pH-shift-Effekt nicht bei einer Auswahl von anionischen bzw. carboxylgruppenhaltigen Polymeren einstellt, deren Glastemperatur nicht mehr als 70 °C beträgt, sondern sich der aufgabengemäße Effekt der Veränderung des zeitlichen Verlaufs der Wirkstofffreisetzungscharakteristik ohne Veränderung des Auflöse-pH-Werts ergibt.

Insbesondere wird die Aufgabe gelöst durch eine
Arzneiform, bestehend aus einem wirkstoffhaltigen Kern, der mit einem polymeren Mischüberzug überzogen ist, dadurch gekennzeichnet, dass der Mischüberzug eine Mischung aus 2 bis 60 Gew.-% eines Polymeren (I) mit 40 bis 98 Gew.-% eines Polymeren (II) ist, wobei der Polymermischung pharmazeutisch übliche Hilfsstoffe beigemengt sein können,
dadurch gekennzeichnet, dass
das Polymer (I) ein Copolymer aus 10 bis 30 Gew.-%, Methylmethacrylat, 50 bis 70 Gew.-% Methylacrylat und 5 bis 15 Gew.-% Methacrylsäure ist und das Polymer (II) ein vom Polymer (I) verschiedenes Vinylpolymer oder ein Polysaccharid oder ein Derivat eines Polysaccharids ist, das sich zu 88 bis 100 % aus neutralen Monomereinheiten zusammensetzt und bis zu 12 Gew.-% Monomereinheiten mit ionischen Resten enthalten kann,
wobei das Polymer II ein Copolymer aus Methylmethacrylat und Ethylacrylat,, ein Copolymer aus Methylmethacrylat, Ethylacrylat und Trimethylammnniumethylmethacrylat, Polyvinylacetat (PVAc), Hydroxyethylcellulose (HEC), Ethylcellulose (EC) oder eine Mischung der genannten Polymere ist,
und ein Wirkstofffreigabeprofil erhalten wird, bei welchem der Wirkstoff im Vergleich zu einer mit dem Polymer (I) allein überzogenen Arzneiform, beginnend beim gleichen pH-Wert jedoch verlangsamt freigesetzt wird.

### Ausführung der Erfindung

### Mischungsverhältnisse Polymer (I) zu Polymer (II)

Die Mischung enthält bzw. besteht im Wesentlichen bzw. bevorzugt zu 100 % aus 2 bis 60, bevorzugt 2 bis 30 Gew.-% eines oder mehreren Polymeren (I) und 40 bis 98, bevorzugt 70 bis 98 Gew.-% eines oder mehreren Polymeren (II). In diesem Bereich können annähernd alle Übergänge zwischen den Freisetzungsprofilen der Polymere (I) und (II) eingestellt werden, so dass den Fachmann eine neue Alternative bei Formulierung von Arzneiformen bereitsteht.

Eine bevorzugte Mischung enthält bzw. besteht im Wesentlichen bzw. bevorzugt zu 100 % aus 2 bis 15 Gew.-% eines oder mehreren Polymeren (I) mit 85 bis 98 Gew.-% einem oder mehreren Polymeren (II). In diesem Bereich wird durch überraschenderweise bereits durch einen verhältnismäßig geringen Anteil des Polymeren (I), die unerwünscht stark verzögernde Freisetzungscharakteristik des Polymers (II) in einem Bereich gelenkt, der für eine lang anhaltende, annähernd konstante Freisetzung einer Vielzahl von Wirkstoffen in den verschiedenen Darmabschnitten aus therapeutischer Sicht wünschenswert ist. Bevorzugt wird der Wirkstoff bei dem pH-Wert, bei dem sich das Polymer (I) aufzulösen beginnt, im Freisetzungstest nach USP (USP 28-NF23) in 60 Minuten zu weniger als 50 % freigesetzt wird. Insbesondere ist es günstig wenn der Wirkstoff bei dem pH-Wert, bei dem sich das Polymer (I) aufzulösen beginnt, im Freisetzungstest nach USP in 60 Minuten zu mehr als 10 % freigesetzt wird.

Dabei wird der Freisetzungsgrad stets auch von der Schichtdicke des Überzugs beeinflusst. Diese kann bei vorgegebenem Mischungsverhältnis erhöht oder erniedrigt werden, um die Freisetzung in gewünschten Bereich zu steuern.

Die Wirkstofffreisetzung kann nach USP, insbesondere USP 28-NF23, General Chapter <711>, *Dissolution,* Apparatus 2 (Paddle), Method <724> "Delayed Release (Enteric Coated) Articles-General General Drug Release Standard", Method B (100 Upm, 37 °C) mit folgender Abwandlung bestimmt werden: Die überzogenen Pellets wurden zunächst für 120 min in künstlichen Magensaft (USP) bei pH 1,2 auf Magensaftresistenz geprüft, anschließend wird mit Phosphatpuffer auf pH 7,5 umgepuffert, was einem künstlichen Darmmillieu entspricht. Die Wirkstoffkonzentration im Testmedium kann abhängig vom Wirkstoff z. B. photometrisch bestimmt werden.

### Polymere (I)

### Glastemperatur

Die Glastemperatur des Polymeren (I) beträgt nicht mehr als 70 °C, bevorzugt 45 bis 68 °C.

Unter Glastemperatur wird hier insbesondere die midpoint temperature *T*_{mg} nach ISO 11357-2, Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

### Zusammensetzung des Polymeren (I)

Die Polymere (I) sind (Meth)acrylat-Copolymere, enthaltend bzw. bestehend zu 90 bis 100, bevorzugt zu 95 bis 100, besonders bevorzugt zu 100 Gew.-% aus 66 bis 95 Gew.-% radikalisch polymerisierten Einheiten von C₁- bis C₄-Alkylestern der Acryl- oder der Methacrylsäure und 5 bis 34 Gew.-% Einheiten von (Meth)acrylat-Monomeren mit einer anionischen Gruppe. Gegebenenfalls können 0 bis 10 Gew.-% Reste aus weiteren vinylisch poylmerisierbaren Monomeren im Polymer (I) enthalten sein.

C₁- bis C₄-Alkylestern der Acryl- oder Methacrylsäure sind insbesondere Methylmethacrylat, Ethylmethacrylat, Butylmethacrylat, Methylacrylat, Ethylacrylat und Butylacrylat.

Ein (Meth)acrylat-Monomer mit einer anionischen Gruppe kann z. B. Acrylsäure, bevorzugt jedoch Methacrylsäure sein.

In der Regel addieren sich die genannten Anteile der C₁- bis C₄-Alkylestern der Acryl- oder der Methacrylsäure und der (Meth)acrylat-Monomere mit einer anionischen Gruppe zu 100 Gew.-%. Die meisten handelsüblichen Polymere (I) enthalten keine Reste weiterer Monomer-Typen auf.

Es können jedoch zusätzlich, ohne dass dies zu einer Beeinträchtigung oder Veränderung der wesentlichen Eigenschaften der Polymere (I) führt, geringe Mengen im Bereich von 0 bis 10, z. B. 1 bis 5 Gew.-% weiterer vinylisch copolymerisierbarer Monomere, wie z. B. Hydroxyethylmethacrylat oder Hydroxyethylacrylat Butylacrylat, Vinylpyrrolidon, Vinylmalonsäure, Styrol, Vinylalkohol, Vinylacetat und/oder deren Derivate enthalten sein. Bevorzugt sind jedoch keine weiteren vinylisch copolymerisierbarer Monomere enthalten.

Die Glastemperatur des Polymeren (I) beträgt nicht mehr als 70, bevorzugt 40 bis 70, besonders bevorzugt 45 bis 65, insbesondere 45 bis 55 °C,.

Unter Glastemperatur wird hier insbesondere die midpoint temperature *T*_{mg} nach ISO 11357-2, Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

### Dispersionen / Teilneutralisation

Das Polymer (I) ist in der Regel ein Emulsionspolymerisat und wird vorzugsweise in Form einer 10- bis 50-gew.-prozentigen, insbesondere 20 bis 40-prozentigen wäßrigen Dispersion erzeugt und angewendet. Als Handelsform ist ein Feststoffgehalt von 30 Gew.-% bevorzugt. Für die Verarbeitung ist eine teilweise Neutralisation der Methacrylsäure-Einheiten entbehrlich; sie ist jedoch, beispielsweise in einem Umfang bis zu 5 oder 10 Mol-% möglich, wenn eine Stabilisierung oder Verdickung der Überzugsmitteldispersion erwünscht sein sollte. Der Gewichtsmittelwert Latex-Teilchengröße (Radius) beträgt in der Regel 40 bis 100 nm, vorzugsweise 50 bis 70 nm, was eine verarbeitungstechnisch günstige Viskosität unter 1000 mPa · s gewährleistet. Die Teilchengröße kann durch Laserbeugung, z. B. mit dem Mastersizer 2000 (Fa. Malvern), bestimmt werden.

Bei höheren Neutralisationsgrades z. B. 10 bis 50 Mol.-% oder vollständiger Neutralisation ist es möglich, das Copolymer in einen gelösten Zustand zu überführen.

Um eine Lösung des anionischen Copolymers herzustellen ist in der Regel eine teilweise oder vollständige Neutralisation der Säuregruppen nötig. Das anionische Copolymer kann z. B. nach und nach in einer Endkonzentration von 1 bis 40 Gew.-% in Wasser eingerührt werden und dabei teilweise oder vollständig neutralisiert werden durch Zugabe einer basischen Substanz wie z. B. NaOH, KOH, Ammoniumhydroxyd oder organische Basen wie z. B. Triethanolamin. Es ist auch möglich ein Pulver des Copolymeren einzusetzen, dem bereits bei seiner Herstellung zum Zweck der (Teil)neutralisation eine Base z. B. NaOH zugesetzt wurde, so daß das Pulver ein bereits (teil)neutralisiertes Polymer ist. Der pH-Wert der Lösung liegt in der Regel über 4, z. B. im Bereich von 4 bis ca. 7. Man kann dabei auch z. B. Mischungen von Batches von voll- oder teilneutralisierten Dispersionen mit nicht neutralisierten Dispersionen vornehmen und in beschriebener Weise weiterverarbeiten, d. h. die Mischung für Überzüge verwenden oder zunächst zu einem Pulver gefrier- oder sprühtrocknen.

Die Dispersion kann z. B. auch in an sich bekannter Weise sprühgetrocknet oder gefriergetrocknet werden und im Form eines redispergierbaren Pulvers bereitgestellt werden (siehe z. B. EP-A 0 262 326). Alternative Verfahren sind die Gefriertrocknung oder Coagulation uns Abquetschen des Wassers in einem Extruder mit anschließender Granulation (siehe z. B. EP-A 0 683 028).

Überraschenderweise wurde gefunden, daß Copolymer-Dispersionen aus sprüh- oder gefriergetrockneten und redispergierten Pulvern eine erhöhte Scherstabilität aufweisen. Dies ist insbesondere beim Sprühauftrag von Vorteil. Dieser Vorteil tritt insbesondere verstärkt hervor wenn das in der Dispersion enthaltene Copolymer zu 2 bis 10, bevorzugt zu 5 bis 7 Mol-% in teilneutralisierter Form vorliegt (bezogen auf die im Copolymer enthaltenen Säuregruppen). Bevorzugt ist zu diesem Zweck die Teilneutalisation mittels Zugabe von NaOH. Bevorzugt ist ein anionischer Emulgator in Menge von 0,1 bis 2 Gew.-% enthalten. Besonders bevorzugt ist Natiumlaurylsulfat als Emulgator.

### Polymer (I)-Typ mit 5 bis 15 Gew.-% Methacrylsäure

Geeignete Polymere (I), bekannt aus EP 0 704 208 A2, sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS). Der pH-Wert des Beginns der spezifischen Wirkstofffreisetzung in Darmsaft oder künstlichem Darmsaft kann mit pH 7,0 angegeben werden. Die Glastemperatur dieses Polymeren (I) beträgt bevorzugt 45 bis 55 °C.

EUDRAGIT® FS ist ein Copolymer aus 25 Gew.-%, Methylmethacrylat, 65 Gew.-% Methylacrylat und 10 Gew.-% Methacrylsäure. EUDRAGIT® FS 30 D ist eine Dispersion enthaltend 30 Gew.-% EUDRAGIT® FS. Die Glastemperatur *T*_{mg} nach ISO 11357-2, Punkt 3.3.3 beträgt ca. 48 °C.

### Polymer (I)-Typ mit 20 bis 34 Gew.-% Methacrylsäure und guten Reißdehnungseigenschften

Weiterhin geeignete Polymere (I) sind Copolymere, bekannt aus WO 03/072087, aus
20 bis 34 Gew.-% Methacrylsäure und/oder Acrylsäure,
20 bis 69 Gew.-% Methylacrylat und
0 bis 40 Gew.-% Ethylacrylat und/oder gegebenenfalls
0 bis 10 Gew.-% weiteren vinylisch copolymerisierbarer Monomeren
mit der Maßgabe, dass die Monomerenanteile so gewählt sind, dass die Glastemperatur des Copolymers nach ISO 11357-2, Punkt 3.3.3, höchstens 60 °C beträgt. Dieses (Meth)acrylatcopolymer ist wegen seiner guten Reißdehungseigenschaften insbesondere zum Verpressen von Pellets zu Tabletten geeignet.

Das oben genannte Copolymer setzt sich insbesondere zusammen aus radikalisch polymerisierten Einheiten von
20 bis 34, bevorzugt 25 bis 33, besonders bevorzugt 28 bis 32 Gew.-% Methacrylsäure oder Acrylsäure, bevorzugt ist Methacrylsäure,
20 bis 69, bevorzugt 35 bis 65, besonders bevorzugt 35 bis 55 Gew.-% Methylacrylat und gegebenenfalls
0 bis 40, bevorzugt 5 bis 35, besonders bevorzugt 15 bis 35 Gew.-% Ethylacrylat zusammen, mit der Maßgabe, dass die Glastemperatur des Copolymers (Messung ohne Weichmacherzusatz bei einem Restmonomergehalt (REMO) von weniger als 100 ppm, Aufheizrate 10 °C/min, Stickstoffatmosphare) nach ISO 11357-2, Punkt 3.3.3 (*T*_{mg}), höchstens 60, bevorzugt 40 bis 60, besonders bevorzugt 45 bis 55 °C beträgt.

Das Copolymer besteht bevorzugt in wesentlichen bis ausschließlich aus den Monomeren Methacrylsäure, Methylacrylat und Ethylacrylat in den oben angegegebenen Mengenanteilen.

Es können jedoch zusätzlich, ohne dass dies zu einer Beeinträchtigung der wesentlichen Eigenschaften führt, geringe Mengen im Bereich von 0 bis 10, z. B. 1 bis 5 Gew.-% weiterer vinylisch copolymerisierbarer Monomere, wie z. B. Methylmethacrylat, Butylmethacrylat, Butylacrylat oder Hydroxyethylmethacrylat enthalten sein.

Zur Einstellung spezieller Freisetzungsprofile bzw. Freisetzungsorte können auch Mischungen der genannten Copolymere zum Einsatz kommen.

Unter Glastemperatur wird hier insbesondere die midpoint temperature *T*_{mg} nach ISO 11357-2, Punkt 3.3.3, verstanden. Die Messung erfolgt ohne Weichmacherzusatz, bei Restmonomergehalten (REMO) von weniger als 100 ppm, bei einer Aufheizrate von 10 °C/min und unter Stickstoffatmosphäre.

Die Copolymere werden in an sich bekannter Weise durch radikalische Substanz-, Lösungs-, Perl- oder Emulsionspolymerisation erhalten. Sie müssen vor der Verarbeitung durch geeignete Mahl-, Trocken- oder Sprühprozesse in den erfindungsgemäßen Teilchengrößenbereich gebracht werden.
Dies kann durch einfaches Brechen extrudierter und abgekühlter Granulatstränge oder Heißabschlag erfolgen.

Insbesondere bei Mischung mit weiteren Pulvern oder Flüssigkeiten kann der Einsatz von Pulvern vorteilhaft sein. Geeignete Gerätschaften zur Herstellung der Pulver sind dem Fachmann geläufig, z. B. Luftstrahlmühlen, Stiftmühlen, Fächermühlen. Gegebenenfalls können entsprechende Siebungsschritte einbezogen werden. Eine geeignete Mühle für industrielle Großmengen ist zum Beispiel eine Gegenstrahlmühle (Multi Nr. 4200), die mit ca. 6 bar Überdruck betrieben wird.

### Polymer (I)-Typ mit 20 bis 33 Gew.-% Methacrylsäure mit guten mechanischen Eigenschaften, insbesondere zum Verpressen von Pellets zu Tabletten.

Weiterhin geeignete Polymere (I) sind Copolymere, bekannt aus WO 2004/096185, aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und
gegebenenfalls
0 bis 10 Gew.-% weiteren vinylisch copolymerisierbarer Monomeren,
wobei sich die Anteile der Monomeren zu 100 Gew.-% addieren,
mit der Maßgabe, dass die Monomerenanteile so gewählt sind, dass die Glastemperatur des Copolymers (glass transition temperature) nach ISO 11357-2, Punkt 3.3.3 (midpoint temperature *T*_{mg}), 55 bis 70 °C beträgt. Copolymere dieses Typs sind wegen ihrer guten mechanischen Eigenschaften insbesondere zum Verpressen von Pellets zu Tabletten geeignet.

Das oben genannte Copolymer setzt sich insbesondere zusammen aus radikalisch polymerisierten Einheiten von
20 bis 33, bevorzugt 25 bis 32, besonders bevorzugt 28 bis 31 Gew.-% Methacrylsäure oder Acrylsäure, bevorzugt ist Methacrylsäure,
5 bis 30, bevorzugt 10 bis 28, besonders bevorzugt 15 bis 25 Gew.-% Methylacrylat,
20 bis 40, bevorzugt 25 bis 35, besonders bevorzugt 18 bis 22 Gew.-% Ethylacrylat, sowie
größer 10 bis 30, bevorzugt 15 bis 25, besonders bevorzugt 18 bis 22 Gew.-% Butylmethacrylat
zusammen, wobei die Monomerzusammensetzung so gewählt wird, daß die Glastemperatur des Copolymers 55 bis 70 °C, bevorzugt 59 bis 66, besonders bevorzugt 60 bis 65 °C beträgt.

Das Copolymer besteht bevorzugt in wesentlichen bis ausschließlich, zu 90, 95 oder 99 bis 100 Gew.-%, aus den Monomeren Methacrylsäure, Methylacrylat, Ethylacrylat und Butylmethacrylat in den oben angegebenen Mengenbereichen.

Es können jedoch zusätzlich, ohne daß dies zu einer Beeinträchtigung der wesentlichen Eigenschaften führen muß, geringe Mengen im Bereich von 0 bis 10, z. B. 1 bis 5 Gew.-% weiterer vinylisch copolymerisierbarer Monomere, wie z. B. Methylmethacrylat, Butylacrylat, Hydroxyethylmethacrylat, Vinylpyrrolidon, Vinylmalonsäure, Styrol, Vinylalkohol, Vinylacetat und/oder deren Derivate enthalten sein.

### Polymere (II)

Das Polymer (II) ist ein vom Polymer (I) verschiedenes Vinylpolymer oder ein Polysaccharid oder ein Derivat eines Polysaccharids, das sich zu 80 bis 100 % aus neutralen Monomereinheiten zusammensetzt und bis zu 12 Gew.-% Monomereinheiten mit ionischen Resten enthalten kann, wobei das Polymere (II) ein Copolymer aus Methylmethacrylat und Ethylacrylat, ein Copolymer aus Methylmethacrylat Ethylacrylat und Trimethylammoniumethylmethacrylat, Polyvinylacetat (PVAc), Hydroxyethylcellulose (HEC), Ethylcellulose (EC) oder eine Mischung der genannten Polymere ist.

Das Polymer (II) kann besonders bevorzugt ein Copolymer aus 20 bis 40 Gew.-% Ethylacrylat und 60 bis 80 Gew.-% Methylmethacrylat sein (Typ EUDRAGIT® NE) sein.

Insbesondere geeignet als Polymer (II) ist ein Copolymer aus 30 Gew.-% Ethylacrylat und 70 Gew.-% Methylmethacrylat (EUDRAGIT® NE).

Das Polymer (II) kann ein Copolymer, aus 50 - 70 Gew.-% Methylmethacrylat, 20 - 40 Gew.-% Ethylacrylat und 12 - 2 Gew.-Trimethylammoniumethylmethacrylat-Chlorid sein (Typ EUDRAGIT® RS/RL).

Ein konkret geeignetes Copolymer enthält 65 Gew.-% Methylmethacrylat, 30 Gew.-% Ethylacrylat und 5 Gew.-% 2-Trimethylammoniumethylmethacrylat-Chlorid aufgebaut sein (EUDRAGIT® RS).
Ein konkret geeignetes Copolymer enthält 60 Gew.-% Methylmethacrylat, 30 Gew.-% Ethylacrylat und 10 Gew.-% 2-Trimethylammoniumethylmethacrylat-Chlorid aufgebaut sein (EUDRAGIT® RL).

Wirkstoffhaltige Pellets können hergestellt werden indem man mittels eines Layeringprozesses Wirkstoff aufbringt. Dazu wird Wirkstoff gemeinsam mit weiteren Hilfsstoffen (Trennmittel, ggf. Weichmacher) homogenisiert und in einem Bindemittel gelöst oder suspendiert. Mittels eines Wirbelschichtverfahrens kann die Flüssigkeit auf Placebopellets oder sonstige geeignete Trägermaterialien aufgebracht werden, wobei das Lösungs- oder Suspendiermittel verdunstet wird (Literatur: International Journal of Pharmaceutics 143, S. 13 - 23*)*. Nach dem Herstellverfahren kann sich ein Trocknungsschritt anschließen. Der Wirkstoff kann in mehreren Schichten aufgebracht werden.

Einige Wirkstoffe, z. B. Acetylsalicylsäure, sind in Form von Wirkstoffkristallen handelsüblich und können in dieser Form anstelle von wirkstoffhaltigen Pellets eingesetzt werden.

Filmüberzüge auf wirkstoffhaltige Pellets werden üblicherweise in Wirbelschichtgeräten aufgebracht. Rezepturbeispiele sind in dieser Anmeldung erwähnt. Filmbildner werden üblicherweise mit Weichmachern und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer Zerfallszeiten kann eine spezielle Reihenfolge bei der Zugabe der Hilfsstoffe zu den überzogenen Partikel erforderlich sein. Durch Vormischung mit der überzogenen Partikel mit dem Schmier- oder Formentrennmittel Magnesiumstearat kann dessen Oberfläche hydrophobisiert und somit Verkleben vermieden werden.

Zum Tablettieren geeignete Mischungen enthalten üblicherweise 3 bis 15 Gew.-% eines Zerfallshilfsmittels, z. B. Kollidon CL und z. B. 0,1 bis 1 Gew.-% eines Schmier- und Formentrennmittels wie Magnesiumstearat. Der Bindemittelanteil bestimmt sich nach dem geforderten Anteil an überzogenen Partikeln.

Typische Bindemittel sind z. B. Cellactose^{®}, mikrokristalline Cellulose, Calciumphosphate, Ludipress^{®}, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier- und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zur Fließverbesserung beigefügt sein (z. B. hochdisperse Kieselsäurederivate, Talkum usw.).

Das Tablettieren kann auf üblichen Tablettenpressen, Exzenter- oder Rundlauftablettenpressen erfolgen, bei Preßkräften im Bereich von 5 bis 40 kN, bevorzugt 10 - 20 kN. Die Tablettenpressen können mit Systemen zur externen Schmierung ausgestattet sein. Gegebenenfalls kommen spezielle Systeme zur Matrizenbefüllung zum Einsatz, die die Matrizenbefüllung mittels Rührflügeln vermeiden.

### Polymermischung

Zunächst wird eine Mischung eines oder mehreren Polymer (I) und eines oder mehreren Polymer (II) hergestellt. Dazu werden z. B. zwei organische Lösungen oder zwei wässrige Dispersionen anteilig gemischt. Bevorzugt wird die Mischung aus wässrigen Dispersionen eines oder mehreren des Polymeren (I) und eines oder mehreren des Polymeren (II) hergestellt. In der Regel wird man jeweils ein Polymer (I) und ein Polymer (II) einsetzen. Die Mischung enthält 2 bis 60, bevorzugt 10 bis 55 Gew.-% eines Polymeren (I) mit 40 bis 98, bevorzugt 45 bis 90 Gew.-% eines oder mehreren Polymeren (II), wobei sich die Anteile zu 100 Gew.-% ergänzen. In der Regel jedoch nicht zwingend sind zusätzlich pharmazeutisch übliche Hilfsstoffe beigemengt, die ggf. separat gelöst oder dispergiert werden.

### Arzneiform

Die Erfindung betrifft weiterhin eine Arzneiform mit einem ausgewählten Polymer (I), das aus EP 0 152 038 A2 nicht hervorgeht. Das in der Arzneiform enthaltene Polymer (II) ist identisch mit den hier beschriebenen verwendungsgemäß eingestezten Polymeren (II).

Die Erfindung betrifft demnach eine Arzneiform, bestehend aus einem wirkstoffhaltigen Kern, der mit einem polymeren Mischüberzug überzogen ist, dadurch gekennzeichnet, dass der Mischüberzug eine Mischung aus 2 bis 60 Gew.-% eines Polymeren (I) mit 40 bis 98 Gew.-% eines Polymeren (II) ist, wobei der Polymermischung pharmazeutisch übliche Hilfsstoffe beigemengt sein können,
dadurch gekennzeichnet, dass
das Polymer (I) ein Copolymer aus 10 bis 30 Gew.-%, Methylmethacrylat, 50 bis 70 Gew.-% Methylacrylat und 5 bis 15 Gew.-% Methacrylsäure ist und das Polymer (II) ein vom Polymer (I) verschiedenes Vinylpolymer oder ein Polysaccharid oder ein Derivat eines Polysaccharids ist, das sich zu 88 bis 100 % aus neutralen Monomereinheiten zusammensetzt und bis zu 12 Gew.-% Monomereinheiten mit ionischen Resten enthalten kann,
wobei das Polymer (II) ein Copolymer aus Methylmethacrylat und Ethlaclat, , ein Copolymer aus Methylmethacrylat, Ethylacrylat und Trimethylammoniumethylmethacrylat, Polyvinylacetat (PVAc), Hydroxyethylcellulose (HEC), Ethylcellulose (EC) oder eine Mischung der genannten Polymere ist,
und ein Wirkstofffreigabeprofil erhalten wird, bei welchem der Wirkstoff im Vergleich zu einer mit dem Polymer (I) allein überzogenen Arzneiform, beginnend beim gleichen pH-Wert jedoch verlangsamt freigesetzt wird.

### Polymer (I)-Typ mit 5 bis 15 Gew.-% Methacrylsäure

Geeignete Polymere (I) für die erfindungsgemäße Arzneiform sind bekannt aus EP 0 704 208 A2. Polymere (I) sind (Meth)acrylat Copolymere, bestehend aus 10 bis 30 Gew.-%, Methylmethacrylat, 50 bis 70 Gew.-% Methylacrylat und 5 bis 15 Gew.-% Methacrylsäure (Typ EUDRAGIT® FS). Der pH-Wert des Beginns der spezifischen Wirkstofffreisetzung in Darmsaft oder künstlichem Darmsaft kann mit pH 7,0 angegeben werden. Die Glastemperatur dieses Polymeren (I) beträgt bevorzugt 45 bis 55 °C.

EUDRAGIT® FS ist ein Copolymer aus 25 Gew.-%, Methylmethacrylat, 65 Gew.-% Methylacrylat und 10 Gew.-% Methacrylsäure. EUDRAGIT® FS 30 D ist eine Dispersion enthaltend 30 Gew.-% EUDRAGIT® FS. Die Glastemperatur *T*_{mg} nach ISO 11357-2, Punkt 3.3.3 beträgt ca. 48 °C.

### Allgemeines Herstellungsverfahren für die beschriebenen Arzneiformen

### Kerne

Träger für die Überzüge sind Kapseln, Tabletten, Granulate, Pellets, Kristalle von regelmäßiger oder unregelmäßiger Form. Die Größe von Granulaten, Pellets oder Kristallen liegt zwischen 0,01 und 2,5 mm, die von Tabletten zwischen 2,5 und 30,0 mm. Kapsel bestehen aus Gelatine, Stärke oder Cellulosederivaten.

Sie enthalten in der Regel die biologisch aktive Substanz (Wirkstoff) bis zu 95 % sowie weitere pharmazeutische Hilfsstoffe bis zu 99,9 Gew.-% Übliche Herstellungsverfahren sind direktes Verpressen, Verpressen von Trocken-, Feucht- oder Sintergranulaten, Extrusion und anschließende Ausrundung, feuchte oder trockene Granulation oder direkte Pelletierung (z.B. auf Tellern) oder durch Binden von Pulvern (Powder layering) auf wirkstofffreie Kugeln (Nonpareilles) oder wirkstoffhaltige Partikeln.

Neben dem Wirkstoff können sie weitere pharmazeutische Hilfsstoffe enthalten: Bindemittel, wie Zellulose und deren Derivate, Polyvinylpyrrolidon (PVP), Feuchthaltemittel, Zerfallsförderer, Gleitmittel, Sprengmittel, (Meth)acrylate, Stärke und deren Derivate, Zucker Solubilisatoren oder andere.

### Herstellung einer Arzneiform

Wirkstoffhaltige Pellets können z. B. hergestellt werden indem man mittels eines Layeringprozesses Wirkstoff aufbringt. Dazu wird Wirkstoff gemeinsam mit weiteren Hilfsstoffen (Trennmittel, ggf. Weichmacher) homogenisiert und in einem Bindemittel gelöst oder suspendiert. Mittels eines Wirbelschichtverfahrens kann die Flüssigkeit auf Placebopellets oder sonstige geeignete Trägermaterialien aufgebracht werden, wobei das Lösungs- oder Suspendiermittel verdunstet wird (Literatur: International Journal of Pharmaceutics 143, S. 13 - 23*)*. Nach dem Herstellverfahren kann sich ein Trocknungsschritt anschließen. Der Wirkstoff kann in mehreren Schichten aufgebracht werden.

Einige Wirkstoffe, z. B. Acetylsalicylsäure, sind in Form von Wirkstoffkristallen handelsüblich und können in dieser Form anstelle von wirkstoffhaltigen Pellets eingesetzt werden.

Zunächst wird ein Mischung des Polymers (I) und des Polymers (II) hergestellt. Dazu werden z. B. zwei Dispersionen anteilig gemischt.

Filmüberzüge auf wirkstoffhaltige Pellets werden üblicherweise in Wirbelschichtgeräten aufgebracht. Rezepturbeispiele sind in dieser Anmeldung erwähnt. Filmbildner werden üblicherweise mit Weichmachern und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

### Polymerüberzug

Der Polymerüberzug kann bevorzugt z. B. 2 bis 20 Gew.-% in Bezug auf das Gewicht des wirkstoffhaltigen Kerns ausmachen. Dabei wird der Freisetzungsgrad stets auch von der Schichtdicke des Überzugs beeinflusst. Diese kann bei vorgegebenem Mischungsverhältnis erhöht oder erniedrigt werden, um die Freisetzung in gewünschten Bereich zu steuern.

### Herstellung multipartikulärer Arzneiformen

Die überzogene Arzneiform liegt bevorzugt in Form von Pellets vor, die in einer multipartikulären Arzneiform, insbesondere in pellethaltigen Tabletten, Minitabletten, Kapseln, Sachets oder Trockensäften enthalten sind.

Die Erfindung eignet sich insbesondere zur Herstellung multipartikulärer Arzneiformen, da die erfindungsgemäße Mischung den hohen Drücken beim Verpressen der Pellets mit dem Füllstoff standhält. Die überzogene Arzneiform liegt bevorzugt in Form von Pellets vor, die in einer multipartikulären Arzneiform, insbesondere in pellethaltigen Tabletten, Minitabletten, Kapseln, Sachets oder Trockensäften enthalten sind.

Die Herstellung von multipartikulären Arzneiformen durch Verpressen eines pharmazeutisch üblichen Bindemittels mit wirkstoffhaltigen Partikeln ist z. B. Beckert et al. (1996), "Compression of enteric-coated pellets to disintegrating tablets", International Journal of Pharmaceutics 143, S. 13 - 23, und in WO 96/01624 ausführlich beschrieben.

Wirkstoffhaltige Pellets können hergestellt werden indem man mittels eines Layeringprozesses Wirkstoff aufbringt. Dazu wird Wirkstoff gemeinsam mit weiteren Hilfsstoffen (Trennmittel, ggf. Weichmacher) homogenisiert und in einem Bindemittel gelöst oder suspendiert. Mittels eines Wirbelschichtverfahrens kann die Flüssigkeit auf Placebopellets oder sonstige geeignete Trägermaterialien aufgebracht werden, wobei das Lösungs- oder Suspendiermittel verdunstet wird (Literatur: International Journal of Pharmaceutics 143, S. 13 - 23). Nach dem Herstellverfahren kann sich ein Trocknungsschritt anschließen. Der Wirkstoff kann in mehreren Schichten aufgebracht werden.

Einige Wirkstoffe, z. B. Acetylsalicylsäure, sind in Form von Wirkstoffkristallen handelsüblich und können in dieser Form anstelle von wirkstoffhaltigen Pellets eingesetzt werden.

Filmüberzüge auf wirkstoffhaltige Pellets werden üblicherweise in Wirbelschichtgeräten aufgebracht. Rezepturbeispiele sind in dieser Anmeldung erwähnt. Filmbildner werden üblicherweise mit Weichmachern und Trennmitteln nach einem geeigneten Verfahren gemischt. Hierbei können die Filmbildner als Lösung oder Suspension vorliegen. Die Hilfsstoffe für die Filmbildung können ebenfalls gelöst oder suspendiert sein. Organische oder wässrige Löse- oder Dispergiermittel können verwendet werden. Zur Stabilisierung der Dispersion können zusätzlich Stabilisatoren verwendet werden (Beispiel: Tween 80 oder andere geeignete Emulgatoren bzw. Stabilisatoren).

Beispiele für Trennmittel sind Glycerolmonostearat oder andere geeignete Fettsäurederivate, Kieselsäurederivate oder Talkum. Beispiele für Weichmacher sind Propylenglykol, Phthalate, Polyethylenglykole, Sebacate oder Citrate, sowie andere in der Literatur erwähnte Substanzen.

Mischungen zur Herstellung von Tabletten aus überzogenen Partikeln werden durch Vermischen der Pellets mit geeigneten Bindemitteln für die Tablettierung, nötigenfalls der Zugabe von zerfallsfördernden Substanzen und nötigenfalls der Zugabe von Schmiermitteln zubereitet. Das Mischen kann in geeigneten Maschinen stattfinden. Ungeeignet sind Mischer, die zu Schäden an den überzogenen Partikeln führen, z. B. Pflugscharmischer. Zur Erzielung geeigneter kurzer Zerfallszeiten kann eine spezielle Reihenfolge bei der Zugabe der Hilfsstoffe zu den überzogenen Partikenl erforderlich sein. Durch Vormischung mit der überzogenen Partikel mit dem Schmier- oder Formentrennmittel Magnesiumstearat kann dessen Oberfläche hydrophobisiert und somit Verkleben vermieden werden.

Zum Tablettieren geeignete Mischungen enthalten üblicherweise 3 bis 15 Gew.-% eines Zerfallshilfsmittels, z. B. Kollidon CL und z. B. 0,1 bis 1 Gew.-% eines Schmier- und Formentrennmittels wie Magnesiumstearat. Der Bindemittelanteil bestimmt sich nach dem geforderten Anteil an überzogenen Partikeln.

Typische Bindemittel sind z. B. Cellactose^{®}, mikrokristalline Cellulose, Calciumphosphate, Ludipress^{®}, Lactose oder andere geeignete Zucker, Calciumsulfate oder Stärkederivate. Bevorzugt werden Substanzen mit geringer Schüttdichte.

Typische Zerfallshilfsmittel (Sprengmittel) sind quervernetzte Stärke- oder Cellulosederivate, sowie quervernetztes Polyvinylpyrrolidon. Ebenso sind Cellulosederivate geeignet. Durch Auswahl eines geeigneten Bindemittels kann die Verwendung von Zerfallshilfsmittel entfallen.

Typische Schmier- und Formentrennmittel sind Magnesiumstearate oder andere geeignete Salze von Fettsäuren oder in der Literatur zu diesem Zweck aufgeführte Substanzen (z.B. Laurinsäure, Calciumstearat, Talkum usw.). Bei Verwendung geeigneter Maschinen (z.B. Tablettenpresse mit externer Schmierung) oder geeigneter Formulierungen kann die Verwendung eines Schmier- und Formentrennmittels in der Mischung entfallen.

Der Mischung kann gegebenenfalls ein Hilfsmittel zur Fließverbesserung beigefügt sein (z. B. hochdisperse Kieselsäurederivate, Talkum usw.).

Das Tablettieren kann auf üblichen Tablettenpressen, Exzenter- oder Rundlauftablettenpressen erfolgen, bei Preßkräften im Bereich von 5 bis 40 kN, bevorzugt 10 - 20 kN. Die Tablettenpressen können mit Systemen zur externen Schmierung ausgestattet sein. Gegebenenfalls kommen spezielle Systeme zur Matrizenbefüllung zum Einsatz, die die Matrizenbefüllung mittels Rührflügeln vermeiden.

### Wirkstofffreisetzung

Es wird erfindungsgemäß ein Wirkstofffreigabeprofil erhalten, bei welchem der Wirkstoff im Vergleich zu einer mit dem Polymer (I) allein überzogenen Arzneiform, beginnend beim gleichen pH-Wert jedoch verlangsamt freigesetzt wird.

Es wird erfindungsgemäß ein Wirkstofffreigabeprofil erhalten, bei welchem der Wirkstoff im Vergleich zu einer mit dem Polymer (II) allein überzogenen Arzneiform, beginnend beim gleichen pH-Wert jedoch schneller freigesetzt wird.

Bevorzugt sind Arzneiformen, bei denen der Wirkstoff bei einem pH-Wert, bei dem sich das Polymer (I) aufzulösen beginnt, im Freisetzungstest nach USP (USP 28-NF23) in 60 Minuten zu weniger als 50 %, bevorzugt zu weniger als 25 %, besonders bevorzugt zu 10 bis 50 % freigesetzt.

Der Freisetzungstest z. B. nach USP (nach USP 28-NF23, Methode B, modifizierter Test für "enteric coated products") ist dem Fachmann bekannt. Die Versuchsbedingungen sind insbesondere: Paddle-Methode, 100 Umdrehungen pro Minute, 37 °C; pH 1,2 mit 0,1 N HCl, pH 7,5 durch Zugabe von 0,2 M Phosphatpuffer und Einstellen mit 2 N NaOH. Siehe auch USP 27-NF22 Supplement 1, Methode "Delayed Release" Monographie <724> Drug Release.

### Pharmazeutisch übliche Hilfsstoffe

Der erfindungsgemäßen Formulierung werden bevorzugt bei der Herstellung der Granulate oder Pulver Pharmazeutisch übliche Hilfsstoffe hinzugefügt. Die Zuschlagstoffe können auch noch bei der Verarbeitung zum Überzugs- und Bindemittel hinzugefügt werden. Grundsätzlich müssen natürlich alle eingesetzten Substanzen toxikologisch unbedenklich und insbesondere in Arzneimitteln ohne Risiko für Patienten zu verwenden sein.

Einsatzmengen und Verwendung der üblichen Zuschlagstoffe in Arzneimittelüberzügen oder Beschichtungen sind dem Fachmann geläufig. Übliche Zuschlagstoffe können z. B. Trennmittel, Pigmente, Stabilisatoren, Antioxidantien, Porenbildner, Penetrationsförderer, Glanzmittel, Aromastoffe oder Geschmacksmittel sein. Sie dienen als Verarbeitungshilfsmittel und sollen ein sicheres und reproduzierbares Herstellungsverfahren sowie gute Langzeitlagerstabilität gewährleisten oder sie erreichen in der Arzneiform zusätzliche vorteilhafte Eigenschaften. Sie werden den Polymerzubereitungen vor der Verarbeitung zugesetzt und können die Permeabilität der Überzüge beeinflussen, was ggf. als zusätzlicher Steuerparameter genutzt werden kann.

### Trennmittel:

Trennmittel besitzen in der Regel lipophile Eigenschaften und werden in der Regel den Sprühsuspensionen zugesetzt. Sie verhindern eine Agglomeration der Kerne während der Befilmung. Bevorzugt werden Talkum, Mg- oder Ca - Stearat, gemahlene Kieselsäure, Kaolin oder nicht ionische Emulgatoren mit einem HLB - Wert zwischen 3 und 8 eingesetzt. Übliche Einsatzmengen für Trennmittel in den erfindungsgemäßen Überzugs- und Bindemitteln liegen zwischen 0,5 bis 100 Gew.-% bezogen auf das Trockengewicht der Dispersion.

### Pigmente:

Mit dem Überzugsmittel unverträgliche Pigmente sind insbesondere solche Pigmente, die wenn sie der (Meth)acrylat-Copolymer-Dispersion direkt zugesetzt werden, z. B. durch Einrühren, in üblichen Anwendungsmengen von z. B. 20 bis 400 Gew.-% bezogen auf das Trockengewicht des (Meth)acrylat-Copolymeren zur Destabilisierung der Dispersion, Koagulation, zu Entmischungserscheinungen oder ähnlich unerwünschten Effekten führen. Weiterhin sind die zu verwendenden Pigmente natürlich nicht toxisch und für pharmazeutische Zwecke geeignet. Siehe dazu z. B. auch: Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980.

Mit dem Überzugsmittel unverträgliche Pigmente können z. B. Aluminiumoxidpigmente sein. Unverträgliche Pigmente sind z. B., Gelborange, Cochenillerotlack, Farbpigmente auf Basis von Aluminiumoxid bzw Azofarbstoffen, Sulfonsäurefarbstoffe, Gelborange S (E110, C.I. 15985, FD&C Yellow 6), Indigocarmin (E132, C.I. 73015, FD&C Blue 2), Tartrazin (E 102, C.I. 19140, FD&C Yellow 5), Ponceau 4R (E 125, C.I. 16255, FD&C Cochineal Red A), Chinolingleb (E 104, C.I. 47005, FD&C Yellow 10), Erythrosin (E127, C.I. 45430, FD&C Red 3), Azorubin (E 122, C.I. 14720, FD&C Carmoisine), Amaranth (E 123, C. I. 16185, FD&C Red 2), Brilliantsäuregrün (E 142, C.I. 44090, FD&C Green S).

Die angegebenen E-Nummern der Pigmente beziehen sich auf eine EU-Nummerierung. Siehe dazu auch "Deutsche Forschungsgemeinschaft, Farbstoffe für Lebensmittel, Harald Boldt Verlag KG, Boppard (1978); Deutsche Lebensmittelrundschau 74, Nr. 4, S. 156 (1978); Arzneimittelfarbstoffverordnung AmFarbV vom 25.08.1980. Die FD&C-Nummern beziehen sich auf die Zulassung in Food, Drugs und Cosmetics durch U.S. Food and Drug Administration (FDA) beschrieben in: U.S. Food and Drug Administration, Center for Food Safety and Applied Nutrition, Office of Cosmetics and Colors: Code of Federal Regulations - Title 21 Color Additive Regulations Part 82, Listing of Certified Provisionally Listed Colors and Specifications (CFR 21 Part 82).

### Weichmacher

Weitere Zuschlagstoffe können auch Weichmacher sein. Übliche Mengen liegen zwischen 0 und 50, bevorzugt 5 bis 20 Gew.-%.

Weichmacher können je nach Typ (lipophil oder hydrophil) und zugesetzter Menge die Funktionalität der Polymerschicht beeinflussen. Weichmacher erreichen durch physikalische Wechselwirkung mit dem Polymeren eine Absenkung der Glasübergangstemperatur und fördern in Abhängigkeit von der zugesetzten Menge die Verfilmung. Geeignete Stoffe haben in der Regel ein Molekulargewicht zwischen 100 und 20.000 und enthalten eine oder mehrere hydrophile Gruppen im Molekül, z. B. Hydroxyl-, Ester- oder Aminogruppen.

Beispiele geeigneter Weichmacher sind Citronensäurealkylester, Glycerinester, Phthalsäurealkylester, Sebacinsäurealkylester, Succroseester, Sorbitanester, Diethylsebacat, Dibutylsebacat und Polyethylenglykole 200 bis 12.000. Bevorzugte Weichmacher sind Triethylcitrat (TEC), Acetyltriethylcitrat (ATEC) und Dibutylsebacat (DBS). Weiterhin zu nennen sind in der Regel bei Raumtemperatur flüssige Ester wie Citrate, Phthalate, Sebacate oder Rizinusöl. Bevorzugt werden Zitronensäure- und Sebacinsäureester verwendet.

Die Zugabe der Weichmacher zur Formulierung kann in bekannter Weise, direkt, in wässriger Lösung oder nach thermischer Vorbehandlung der Mischung vorgenommen werden. Auch können Mischungen von Weichmachern eingesetzt werden.

### Wirkstoffe

Gebräuchliche Arzneistoffe sind in Nachschlagewerken, wie z.B. der Roten Liste oder dem Merck Index zu entnehmen.

### Biologisch aktive Substanzen:

Die im Sinne der Erfindung eingesetzten Arzneistoffe sind dazu bestimmt, am oder im menschlichen oder tierischen Körper Anwendung zu finden, um
1. Krankheiten, Leiden, Körperschäden oder krankhafte Beschwerden zu heilen, zu lindern, zu verhüten oder zu erkennen.
2. die Beschaffenheit, den Zustand oder die Funktionen des Körpers oder seelische Zustände erkennen lassen.
3. vom menschlichen oder tierischen Körper erzeugte Wirkstoffe oder Körperflüssigkeiten zu ersetzen.
4. Krankheitserreger, Parasiten oder körperfremde Stoffe abzuwehren, zu beseitigen oder unschädlich zu machen oder
5. die Beschaffenheit, den Zustand oder die Funktionen des Körpers oder seelische Zustände zu beeinflussen.

Die erfindungsgemäße Formulierung eignet sich zur Verabreichung grundsätzlich beliebiger pharmazeutischer Wirkstoffe oder biologisch aktiver Substanzen.

### Therapieklassen

Diese pharmazeutisch aktiven Substanzen können einer oder mehrerer Wirkstoffklassen angehören, wie ACE-Hemmer, Adrenergika, Adrenocortikosteroide, Aknetherapeutika, Aldose-Reduktase-Hemmer, Aldosteron-Antagonisten, Alpha-Glucosidasehemmer, Alpha 1- Antagonisten, Mittel gegen Alkoholabusus, Aminosäuren, Amöbizide, Anabolika, Analeptika, Anaesthetika-Zusätze, Anaesthetika (nicht inhalativ), Anaesthetika (lokal), Analgetika, Androgene, Anginatherapeutika, Antagonisten, Antiallergika, Antiallergika wie PDE-Hemmer, Antiallergika zur Asthmabehandlung, Weitere Antiallergika (z.B. Leukotrienantagonisten, Antianämika, Antiandrogene, Antianxiolytika, Antiarthritika, Antiarrhythmika, Antiatheriosklerotika, Antibiotika, Anticholinergika, Anticonvulsiva, Antidepressiva, Antidiabetika, Antidiarrhoika, Antidiuretika, Antidots, Antiemetika, Antiepileptika, Antifibrinolytika, Antiepileptika, Antihelmintika, Antihistaminika, Antihypotensiva, Antihypertensiva, Antihypertonika, Antihypotonika, Antikoagulantien, Antimykotika, Antiöstrogene, Antiöstrogene (Nicht-Steroide), Antiparkinson-Mittel, Antiphlogistika, Antiproliferative Wirkstoffe, Antiprotozoen Wirkstoffe, Antirheumatika, Antischistosomizide, Antispasmolytika, Antithrombotika, Antitussiva, Appetitzügler, Arteriosklerosemittel, Bakteriostatika, Betablocker, Betarezeptorenblocker, Bronchodilatoren, Carboanhydrase-Hemmer, Chemotherapeutika, Choleretika, Cholinergika, Cholinergische Agonisten, Cholinesterase-Hemmer, Mittel zur Behandlung von Colitis ulcerosa, Cyclooxigenasehemmer, Diuretika, Ektoparasitizide, Emetika, Enzyme, Enzym-Hemmer, Enzyminhibitoren, Wirkstoffe gegen Erbrechen, Fibrinolytika, Fungistatika, Gabapentin, Gichtmittel, Glaukomtherapeutika, Glucocorticoide, Glucocortikosteroide, Hämostatika, Herzglykoside, Histamin H2-Antagonisten, Hormone und deren Hemmstoffe, Immuntherapeutika, Kardiotonika, Kokkidiostatika, Laxantien, Lipidsenker, Magen-Darmtherapeutika, Malariatherapeutika, Migränemittel, Mikrobiozide, Morbus Crohn, Metastasenhemmer, Migränemittel, Mineralstoffpräparate, motilitätssteigernde Wirkstoffe, Muskelrelaxantien, Neuroleptika, Wirkstoffe zur Behandlung der Oestrogene, Osteoporose, Otologika, Parkinsonmittel, Phytopharmaka, Pitavastatin, Protonenpumpenhemmer, Prostaglandine, Wirkstoffe zur Behandlung der benignen Prostatahyperblasie, Wirkstoffe zur Behandlung des Pruritus, Psoriasis Wirkstoffe, Psychopharmaka, Radikalfänger, Renin-Antagonisten, Schilddrüsentherapeutika, Wirkstoffe zur Behandlung von Seborrhoe, Wirkstoffe gegen Seekrankheit, Spasmolytika, alpha- und beta-Sympatomimetika, Tenatoprazol, Thrombozytenaggregationshemmer, Tyrosinkinaseinhibitoren, Tranquilizer, Ulkustherapeutika, Weitere Ulkustherapeutika, Mittel zur Behandlung der Urolithiasis, Virustatika, Virustatika, Vitamine, Zytokine, Wirkstoffe für die Kombinationstherapie mit Zytostatika, Zytostatika.

### Wirkstoffe

Beispiele geeigneter Wirkstoffe sind Acarbose, Acetylsalicylsäure, Abacavir, Aceclofenac, Aclarubicin, Acyclovir, Actinomycin, Adalimumab, Adefovir, Adefovirdipivoxil, Adenosylmethionin, Adrenalin und Adrenalinderivate, Agalsidase alpha, Agalsidase beta, Alemtuzumab, Almotriptan, Alphacept, Allopurinol, Almotriptan, Alosetron, Alprostadil, Amantadin, Ambroxol, Amisulprid, Amlodipin, Amoxicillin, 5-Aminosalicylsäure, Amitriptylin, Amlodipin, Amoxicillin, Amprenavir, Anagrelid, Anakinra, Anastrozol, Androgen und Androgenderivate, Apomorphin, Aripiprazol, Arsentrioxid, Artemether, Atenolol, Atorvastatin, Atosiban, Azathioprin, Azelainsäure, Barbitursäurederivate, Balsalazid, Basiliximab, Beclapermin, Beclomethason, Bemiparin, Benzodiazepine, Betahistin, Bexaroten. Bezafibrat, Bicalutamid, Bimatoprost, Bosentan, Botulinumtoxim, Brimonidin, Brinzolamid, Budesonid, Budipin, Bufexamac, Bumetanid, Buprenorphin, Bupropion, Butizin, Calcitonin, Calciumantagonisten, Calciumsalze, Candesartan, Capecitabin, Captopril, Carbamazepin, Carifenacin, Carvedilol, Caspofungin, Cefaclor, Cefadroxil, Cefalexin Cefalosporine, Cefditoren, Cefprozil, Cefuroxim, Celecoxib, Cepecitabin, Cerivastatim, Cetirizin, Cetrorelix, Cetuximab, Chenodeoxycholsäure, Choriogonadotropin, Ciclosporin, Cidofovir, Cimetidin, Ciprofloxacin, Cisplatin, Cladribin, Clarithromycin, Clavulansäure, Clindamycin, Clobutinol, Clonidin, Clopidogrel, Codein, Coffein, Colestyramin, Cromoglicinsäure, Cotrimoxazol, Cumarin und Cumarinderivate, Darbepoetin, Cysteamin, Cystein, Cytarabin, Cyclophosphamid, Cyproteron, Cytarabin, Daclizumab, Dalfopristin, Danaparoid, Dapiprazol, Darbepoetin, Defepripron, Desipramin, Desirudin, Desloaratadin, Desmopressin, Desogestrel, Desonid, Dexibuprofen, Dexketoprofen, Disoproxil, Diazepam und Diazepamderivate, Didanosin, Dihydralazin, Diltiazem, Dimenhydrinat, Dimethylsulfoxid, Dimeticon, Dipivoxil, Dipyridarnoi, Dolasetron, Domperidon und Domperidanderivate, Donepzil, Dopamin, Doxazosin, Doxorubizin, Doxylamin, Diclofenac, Divalproex, Dronabinol, Drospirenon, Drotrecogin alpha, Dutasterid, Ebastin, Econazol, Efavirenz, Eletripan, Emidastin, Emtricitabin, Enalapril, Encepur, Entacapon, Enfurvirtid, Ephedrin, Epinephrin, Eplerenon, Epoetin und Epoetinderivate, Eprosartan, Eptifibatid, Ertapenem, Esomeprazol, Estrogen und Estrogenderivate, Etanercept, Ethenzamid, Ethinöstradiol, Etofenamat, Etofibrat, Etofyllin, Etonogestrel, Etoposid, Exemestan, Ezetimib, Famciclovir, Famotidin, Faropenandaloxat, Felodipin, Fenofibrat, Fentanyl, Fenticonazol, Fexofenadin, Finasterid, Fluconazol, Fludarabin, Flunarizin, Fluorouracil, Fluoxetin, Flurbiprofen, Flupirtin, Flutamid, Fluvastatin, Follitropin, Fomivirsen, Fondaparinux, Formoterol, Fosfomicin, Frovatriptan, Furosemid, Fusidinsäure, Gadobenat, Galantamin, Gallopamil, Ganciclovir, Ganirelix, Gatifloxacin, Gefitinib, Gemfibrozil, Gemopatrilat, Gentamicin, Gepiron, Gestagen und Gestagenderivate, Ginkgo, Glatiramer, Glibenclamid, Glipizide, Glucagon, Glucitol und Glucitolclerivate, Glucosamin und Glucosaminderivate, Glykosidantibiotika, , Glutathion, Glycerol und Glycerolderivate, Hypothalamushormone, Goserelin, Grepafloxacin, Gyrasehemmer, Guanethidin, Gyrasehemmer, Hämin, Halofantrin, Haloperidol, Harnstoffderivate als orale Antidiabetika, Heparin und Heparinderivate, Herzglykoside, Hyaluronsäure, Hydralazin, Hydrochlorothiazid und Hydrochlorothiazidderivate, Hydroxyomeprazol, Hydroxyzin, Ibritumomab, Ibuprofen, Idarubicin, Ifliximab, Ifosfamid, Iloprost, Imatinib, Imidapril, Imiglucerase, Imipramin, Imiquimod, Imidapril, Indometacin, Indoramin, Infliximab, Insulin, Insulin glargin, Interferone, Irbesartan, Irinotecan, Isoconazol, Isoprenalin, Itraconazol, Ivabradine, Jod und Jodderivate, Johanniskraut, Kaliumsalze, Ketoconazol, Ketoprofen, Ketotifen, Lacidipin, Lamotrigin, Lansoprazol, Laronidase, Latanoprost, Leflunomid, Leminoprazol, Lepirudin, Lercanidipin, Leteprinim, Letrozol, Levacetylmethadol, Levetiracetam, Levocetirizin, Levodopa, Levodrpropicin, Levofloxacin, Levomethadon, Licofelone, Linezolid, Lipinavir, Liponsäure und Liponsäurederivate, Lisinopril, Lisurid, Lofepramin, Lodoxamid, Lomefloxacin, Lomustin, Loperamid, Lopinavir, Loratadin, Lornoxicam, Losartan, Lumefantrin, Lutropine, Magnesiumsalze, Makrolidantibiotika, Mangafodipir, Maprotilin, Mebendazol, Mebeverin, Meclozin, Mefenaminsäure, Mefloquin, Meloxicam, Memantin, Mepindolol, Meprobamat,Meropenem, Mesalazin, Mesuximid, Metamizol, Metformin, Methadon, Methotrexat, Methyl-(5-amino-4-oxopentanoat), Methylnaloxon, Methylnaltrexone, Methylphenidat, Methylprednisolon, Metixen, Metoclopramid, Metoprolol, Metronidazol, Mianserin, Mibefradil, Miconazol, Mifepriston, Miglitol, Miglustad, Milnacipran, Minocyclin, Minoxidil, Misoprostol, Mitomycin, Mizolastin, Modafinil, Moexipril, Montelukast, Moroctocog, Morphinane, Morphin und Morphinderivate, Moxifloxacin, Mutterkornalkaloide, Nalbuphin, Naloxon, Naproxen, Naratriptan, Narcotin, Natamycin, Nateglinid, Nebivolol, Nefazodon, Nelfinavir, Neostigmin, Neramexan, Nevirapin, Nicergolin, Nicethamid, Nifedipin, Nifluminsäure, Nimodipin, Nimorazol, Nimustin, Nesiritid, Nisoldipin, Norfloxacin, Novaminsulfon, Noscapin, Nystatin, Ofloxacin, Oktotride, Olanzapin, Olmesartan, Olsalazin, Oseltamivir, Omapatrilat, Omeprazol, Omoconazol, Ondansetron, Orlistat, Oseltamivir, Oxaceprol, Oxacillin, Oxaliplatin, Oxaprozin, Oxcarbacepin, Oxicodon, Oxiconazol, Oxymetazolin, Palivizumab, Palonosetron, Pantoprazol, Paracetamol, Parecoxib, Paroxetin, Pegaspargase, Peg-Interferon, Pegfilgrastrim, Penciclovir, orale Penicilline, Pentazocin, Pentifyllin, Pentoxifyllin, Peptidantibiotika, Perindopril, Perphenazin, Pethidin, Pflanzenextrakte, Phenazon, Pheniramin, Phenylbuttersäure, Phenytoin, Phenothiazine, Phenserin, Phenylbutazon, Phenytoin, Pimecrolimus, Pimozid, Pindolol, Pioglitazon, Piperazin, Piracetam, Pirenzepin, Piribedil, Pirlindol, Piroxicam, Posaconazole, Pramipexol, Pramlintide, Pravastatin, Prazosin, Procain, Promazin, Propiverin, Propranolol, Propionsaurederivate, Propyphenazon, Prostaglandine, Protionamid, Proxyphyllin, Quetiapin, Quinapril, Quinaprilat, Quinupristin, Ramipril, Ranitidin, Rabeprazol, Raloxifen, Ranolazine, Rasburicase, Reboxetin, Repaclinide, Reproterol, Reserpin, Revofloxacin, Ribavirin, Rifampicin, Riluzole, Rimexolon, Risedronat, Risperidon, Ritonavir, Rituximab, Rivastimen, Risatriptan, Rofecoxib, Ropinirol, Ropivacain, Rosiglitazon, Rotigotine, Roxatidin, Roxithromycin, Ruscogenin, Rosuvastatin, Rutosid und Rutosidderivate, Sabadilla, Salbutamol, Salicylate, Salmeterol, Saperconazole, Schilddrüsenhormone, Scopolamin, Selegilin, Sertaconazol, Sertindol, Sertralin, Sevelamer, Sibutramin, Sildenafil, Silikate, Simvastatin, Sirolimus, Sitosterin, Sotalol, Spagluminsäure, Sparfloxacin, Spectinomycin, Spiramycin, Spirapril, Spironolacton, Stavudin, Streptomycin, Sucralfat, Sufentanil, Sulbactam, Sulfonamide, Sulfasalazin, Sulpirid, Sultamicillin, Sultiam, Sumatriptan, Suxamethoniumchlorid, Tacrin, Tacrolimus, Tadalafil, Taliolol, Talsaclidin, Tamoxifen, Tamsulosin, Tasonermin, Tazaroten, Tegafur, Tegaserod, Telithromycin, Telmisartan, Temoporfin, Temozolomid, Tenatoprazol, Tenecteplase, Teniposid, Tenofovir, Tenoxicam, Teriparatid, Terazosin, Terbinafin, Terbutalin, Terfenadin, Teriparatid, Terlipressin, Tertatolol, Testosteron und Testosteronderivate, Tetracycline, Tetryzolin, Tezosentan, Theobromin, Theophyllin, Theophyllinderivate, Thiamazol, Thiotepa, Thr. Wachstumsfaktoren, Tiagabin, Tiaprid, Tibolon, Ticlopidin, Tilidin, Timolol, Tinidazol, Tioconazol, Tioguanin, Tiotropium, Tioxolon, Tirazetam, Tiropramid, Trofiban, Tizanidin, Tolazolin, Tolbutamid, Tolcapon, Tolnaftat, Tolperison, Tolterodin, Topiramat, Topotecan, Torasemid, Tramadol, Tramazolin, Trandolapril, Tranylcypromin, Trapidil, Trastuzumab, Travoprost, Trazodon, Trepostinil, Triamcinolon und Triamcinolonderivate, Triamteren, Trifluperidol, Trifluridin, Trimetazidine, Trimethoprim, Trimipramin, Tripelennamin, Triprolidin, Trifosfamid, Tromantadin, Trometamol, Tropalpin, Trovafloxacin, Troxerutin, Tulobuterol, Trypsine, Tyramin, Tyrothricin, Urapidil, Ursodeoxycholsäure, Theophyllin Ursodeoxycholsäure, Valaciclovir, Valdecoxib, Valganciclovir, Valproinsäure, Valsartan, Vancomycin, Vardenafil, Vecuroniumchlorid, Venlafaxin, Verapamil, Verteporfin, Vidarabin, Vigabatrin, Viloxazin, Vinblastin, Vincamin, Vincristin, Vindesin, Vinorelbin, Vinpocetin, Viquidil, Vitamin D und Derivate von Vitamin D, Voriconazol, Warfarin, Xantinolnicotinat, Ximelagatran, Xipamid, Zafirlukast, Zalcitabin, Zaleplon, Zanamivir, Zidovudin, Ziprasidon, Zoledronsäure, Zolmitriptan, Zolpidem, Zoplicon, Zotepin und dergleichen.

### Besonders bevorzugte Wirkstoffe

Bevorzugte Wirkstoffgruppen sind Analgetika, Antibiotika, Antidiabetika, Antikörper, Peptide, Proteine, Chemotherapeutika, Corticoide/Corticosteroide Entzündungshemmende Mittel, Enzympräparate
Hormone und deren Hemmstoffe, Nebenschilddrüsenhormone Verdauungsfördernde Mittel, Laxantien, Vitamine, Zytostatika sowie Wirkstoffe anderer Gruppen, die aus kinetischen Gründen vorteilhaft in tieferen Darmabschnitten appliziert werden.

Beispiele besonders bevorzugter Wirkstoffe sind Mesalazin, Sulfasalazin, Bethamethason-21-dihydrogenophosphat, Hydrocortison-21-acetat, Cromoglicinsäure, Dexamethason, Olsalazin-Na, Budesonid, Prednison Bismunitrat, Karaya Gummi, Methylprednisolon-21-hydrogensuccinat Myhrre, Kaffeekohle, Kamillenblüttenextrakt, Präparationen von Humanplacenta

Neuere Wirkstoffe sind aus der Literatur oder aus einschlägigen, dem Fachmann bekannten pharmazeutischen Datenbanken zu entnehmen:
Balsalazid ,Adalimumab, Alemtuzumab, Basiliximab, Daclizumab, Ibritumomab, Ifliximab, Cetuximab, Palivizumab, Rituximab, Trastuzumab, andere oral verabreichte Peptide (z.B. RDP 58), Interleukin 6, Interleukin 12, Ilodecakin (Interleukin 10), Nicotintartrat, 5-ASA Konjugate (CPR 2015), Monoclonaler Antikörper gegen Interleukin 12, Diethyldihydroxyhomospermin (DEHOHO), Diethylhomospermin (DEHOP), Cholecystokinin (CCK) Antagonist (CR 1795), 15 Aminosäure-Fragment eines 40 kd Peptids aus Magensaft (BPC 15), Glucocorticoidanalogon (CBP 1011), Natalizumab, Infliximab (REMICADE) N-de-Acetyliertes Lysoglycosphingolipid (WILD 20), Azelastine, Tranilast, Sudismase, Phosphorothioat Antisensoligonucleotid (ISIS 2302), Tazofelone Ropivacaine, 5 Lipoxygenaseinhibitor (A 69412), Sucralfat Die Wirkstoffe können gewünschtenfalls auch in Form ihrer pharmazeutisch annehmbaren Salze oder Derivate verwendet werden, und im Falle chiraler Wirkstoffe können sowohl optisch aktive Isomere als auch Racemate oder Diastereoisomerengemische eingesetzt werden. Die Wirkstoffe können ebenso als physikatische oder chemische Konjugate vorliegen (Polymer-Drug-Conjugates, z. B. Peptid/Protein-Wirkstoffkomplexe). Gewünschtenfalls können die erfindungsgemässen Zusammensetzungen auch zwei oder mehrere pharmazeutische Wirkstoffe enthalten.

### BEISPIELE

### Versuchsbeschreibung

### Gerätschaften

Wirbelschichtgerät Hüttlin Mycrolab
Düse: Dreistoffdüse, Düsendurchmesser: 0,8 mm
Verfahren: Bottomspray
Schlauchpumpe: Ismatec MCP

### Überzüge (Coating)

### Material

Theophyllin-Pellets (Teilchendurchmesser: 0,8 - 1,2 mm)
Wirkstoffgehalt: ca. 93%
Ansatzgröße: 200 bzw. 800g

### Coatingbedingungen

Eingangstemperatur: 33 - 43°C
Prozeßtemperatur: 25 - 31 °C
Sprühdruck: 0,6 - 0,75 bar
Mikroklima: 0,4 - 0,5 bar
Sprührate: bei 200g Ansatzgröße: ca. 12 g/min/kg bei 800g Ansatzgröße: ca. 5 g/min/kg
Probennahme bei 6 und 10% Polymerauftrag.

### Polymere

### Polymertyp (I)

Eudragit® FS 30 D (FS30 D): Methylacrylat Methylmethacrylat Methacrylsäure Copolymer

### Polymertypen (II)

Eudragit® NE 30 D (NE30 D): Ethylacrylat Methylmethacrylat Copolymer
Kollicoat® SR 30 D : Polyvinylacetat
Aquacoat® ECD : Ethylcellulose Polymer
(Alles 30%ige wässrige Dispersionen) Weichmacher : DBS = Dibutylsebacat

### Mischungen

| Polymer | Anteil | | | | | | | | Weichmacher |
|---|---|---|---|---|---|---|---|---|---|
| Eudragit® FS 30 D | 100 | 5 | 10 | 20 | 50 | - | 10 | 10 | - |
| Eudragit® NE 30 D | - | 95 | 90 | 80 | 50 | 100 | - | - | - |
| Kollicoat® SR 30 D | - | - | - | - | _ | - | 90 | - | - |
| Aquacoat® ECD | - | - | - | - | _ | - | - | 90 | 24% DBS bez. a. Polym. |

### Formulierung

### Beispiele

Sprühsuspension für 800g Pellets und 15% Polymerauftragsmenge:

| | Suspension [g] | Feststoff [g] | Anteil [%] |
|---|---|---|---|
| Polymermischung | 400 | 120 | 93,4 |
| Gycerinmonostearat | 6 | 6 | 4,7 |
| Polysorbat 80 | 7,3 | 2,4 | 1,9 |
| VE-Wasser | 228,7 | - | - |
| | 642,0 | 128,4 | 100 |

TS-Gehalt Sprühsuspension: 20,0 %

### Herstellung der Sprühsuspension:

VE-Wasser und Polysorbat 80 werden unter leichtem Rühren auf 75°C erhitzt. Hierzu wird das Glycerinmonostearat gegeben und unter starkem Rühren ca. 30 Minuten homogenisiert. Nach dem Abkühlen auf Raumtemperatur erfolgt die Zugabe der Polymerdispersionen und des Weichmachers. In Bedarfsfällen wird eine Koagulatbildung beim Mischen der Dispersionen durch vorherigen Angleich der pH-Werte vermieden.

### Wirkstofffreigabe (Tabellen)

### Freisetzungstest nach USP

Der Freisetzungstest erfolgt nach USP 28-NF23, General Chapter <711>, *Dissolution,* Apparatus 2 (Paddle), Method <724> "Delayed Release (Enteric Coated) Articles-General General Drug Release Standard", Method B (100 Upm, 37 °C) mit folgender Abwandlung: Die überzogenen Pellets wurden zunächst für 120 min in künstlichen Magensaft (USP) bei pH 1,2 auf Magensaftresistenz geprüft, anschließend wurde mit Phosphatpuffer auf pH 7,5 umgepuffert, was einem künstlichen Darmmillieu entspricht. Die Wirkstoffkonzentration im Testmedium wurde photometrisch bestimmt.

Nach 120 min soll nicht mehr als ca. 5 % des Wirkstoffs freigesetzt sein. Nach 180 min, entsprechend 60 min bei pH 7,5 wird ein Wirkstofffreisetzungsgrad von 5 bis 95 %, bevorzugt von 10 bis 50 % angestrebt.

Die Ergebnisse sind in den Tabellen 1 bis 3 zusammengestellt. Die Angabe 6, 10 und 15 % gibt jeweils das Trockengewicht des Überzugs bezogen auf den Kerngewicht an.

**Tabelle 1**

| Zeit [Min.] | Wirkstofffreigabe [%] | | | | | | | | |
|---|---|---|---|---|---|---|---|---|---|
| | NE 30 D | | | FS 30 D / NE 30 D 5:95 | | | FS 30 D / NE 30 D 10:90 | | |
| | nicht erfindungsgemäß | | | erfindungsgemäß | | | erfindungsgemäß | | |
| | 6% | 10% | 15% | 6% | 10% | 15% | 6% | 10% | 15% |
| 0 | 0,00 | 0,00 | 0,00 | 0,00 | 0,00 | 0,00 | 0,01 | 0,00 | 0,00 |
| 5 | 0,02 | 0,00 | 0,00 | 0,05 | 0,01 | 0,02 | 0,19 | 0,01 | 0,01 |
| 30 | 0,21 | 0,04 | 0,03 | 0,45 | 0,11 | 0,08 | 0,56 | 0,17 | 0,04 |
| 60 | 0,62 | 0,11 | 0,04 | 1,25 | 0,28 | 0,22 | 1,25 | 0,59 | 0,09 |
| 90 | 1,16 | 0,18 | 0,06 | 2,48 | 0,51 | 0,38 | 1,95 | 1,13 | 0,16 |
| **120** | **1,75** | **0,29** | **0,09** | **5,94** | **0,78** | **0,56** | **2,70** | **1,71** | **0,25** |
| 140 | 6,51 | 5,22 | 0,42 | 20,23 | 6,73 | 1,49 | 9,47 | 6,77 | 1,23 |
| 150 | 6,70 | 5,26 | 0,43 | 23,70 | 8,24 | 2,30 | 14,31 | 10,87 | 2,66 |
| 165 | 6,99 | 5,31 | 0,45 | 20,73 | 10,63 | 3,80 | 21,69 | 16,96 | 5,66 |
| **180** | **7,28** | **5,38** | **0,47** | **25,60** | **13,00** | **5,42** | **29,11** | **22,89** | **9,31** |
| 210 | 7,89 | 5,51 | 0,50 | 34,97 | 17,60 | 8,88 | 43,13 | 34,32 | 17,33 |
| 240 | 8,53 | 5,65 | 0,54 | 44,66 | 21,96 | 12,49 | 54,07 | 45,24 | 24,44 |
| 300 | 9,89 | 5,99 | 0,63 | 62,16 | 30,26 | 19,70 | 65,34 | 65,78 | 36,86 |
| 330 | 10,65 | 6,17 | 0,69 | 70,30 | 34,25 | 23,13 | 68,94 | 75,16 | 42,61 |

**Tabelle 2**

| Zeit [Min.] | Wirkstofffreigabe [%] | | | | | | | | |
|---|---|---|---|---|---|---|---|---|---|
| | FS 30 D / NE 30 D 20:80 | | | FS 30 D / NE 30 D 50:50 | | | FS 30 D | | |
| | erfindungsgemäß | | | erfindungsgemäß | | | nicht erfindungsgemäß | | |
| | 6% | 10% | 15% | 6% | 10% | 15% | 6% | | 15% |
| 0 | 0,00 | 0,00 | 0,00 | 0,00 | 0,00 | 0,00 | 0.01 | 0,00 | 0,00 |
| 5 | 0,01 | 0,01 | 0,01 | 0,00 | 0,00 | 0,00 | 0,08 | 0,01 | 0,00 |
| 30 | 0,44 | 0,11 | 0,05 | 0,43 | 0,08 | 0,01 | 0,65 | 0,015 | 0,02 |
| 60 | 1,55 | 0,50 | 0,13 | 1,84 | 0,60 | 0,10 | 1,56 | 0,12 | 0,03 |
| 90 | 2,75 | 0,98 | 0,25 | 3,42 | 1,28 | 0,27 | 2,68 | 0,19 | 0,04 |
| **120** | **3,97** | **1,50** | **0,39** | **5,00** | **1,99** | **0,45** | **3,83** | **0,28** | **0,05** |
| 140 | 45,76 | 34,02 | 22,62 | 51,47 | 44,55 | 25,86 | 100,06 | 100,22 | 98,55 |
| 150 | 64,35 | 48,38 | 36,39 | 70,45 | 63,14 | 44,27 | 99,73 | 99,75 | 99,81 |
| 165 | 84,84 | 66,84 | 53,92 | 87,02 | 82,91 | 66,37 | 99,75 | 99,90 | 99,85 |
| **180** | **94,87** | **81,98** | **69,14** | **94,01** | **92,57** | **81,57** | **99,75** | **99,95** | **99,76** |
| 210 | 97,49 | 97,10 | 91,23 | 96,70 | 99,40 | 97,02 | 99,78 | 99,90 | 99,85 |
| 240 | 97,76 | 98,85 | 97,48 | 96,79 | 99,86 | 99,25 | 99,88 | 99,97 | 99,89 |
| 300 | 97,90 | 99,48 | 99,72 | 96,99 | 99,72 | 99,33 | 100,08 | 100,15 | 99,99 |
| 330 | 98,03 | 99,50 | 99,81 | 97,11 | 99,77 | 99,49 | 100,15 | 100,11 | 100,08 |

**Tabelle 3**

| Zeit [Min.] | Wirkstofffreigabe [%] | | | | | |
|---|---|---|---|---|---|---|
| | FS 30 D / Kollicoat® SR 30 D 10:90 | | | FS 30 D / Aquacoat® ECD 10:90 | | |
| | erfindungsgemäß | | | erfindungsgemäß | | |
| | 6% | 10% | 15% | 6% | 10% | 15% |
| 0 | 0,00 | 0,00 | 0,00 | 0,00 | 0,01 | 0,01 |
| 5 | 0,18 | 0,05 | 0,02 | 0,14 | 0,07 | 0,09 |
| 30 | 1,52 | 0,31 | 0,09 | 1,11 | 0,63 | 0,78 |
| 60 | 3,15 | 0,66 | 0,19 | 2,38 | 1,22 | 1,39 |
| 90 | 4,85 | 1,15 | 0,28 | 3,64 | 1,81 | 2,00 |
| **120** | **6,60** | **1,73** | **0,39** | **5,10** | **2,41** | **2,59** |
| 140 | 13,97 | 2,92 | 0,80 | 5,72 | 2,59 | 2,73 |
| 150 | 22,03 | 6,07 | 0,87 | 6,21 | 2,79 | 2,90 |
| 165 | 35,45 | 13,90 | 1,04 | 7,01 | 3,10 | 3,19 |
| **180** | **49,02** | **23,37** | **1,50** | **7,87** | **3,44** | **3,50** |
| 210 | 73,65 | 43,52 | 6,99 | 9,76 | 4,26 | 4,19 |
| 240 | 91,51 | 63,15 | 18,88 | 11,93 | 5,26 | 5,00 |
| 300 | 99,94 | 93,76 | 49,70 | 16,95 | 7,91 | 7,24 |
| 330 | 100,06 | 98,40 | 65,07 | 19,63 | 9,51 | 8,66 |

## Patentansprüche

1. Verwendung einer Mischung aus 2 bis 60 Gew.-% eines oder mehreren Polymeren (I) mit 40 bis 98 Gew.-% eines oder mehreren Polymeren (II), wobei
das Polymer (I) ein (Meth)acrylat-Copolymer ist, enthaltend 90 bis 100 Gew.-% radikalisch polymerisierte Einheiten aus 66 bis 95 Gew.-% von C₁- bis C₄-Alkylestern der Acryl- oder der Methacrylsäure und 5 bis 34 Gew.-% Einheiten von (Meth)acrylat-Monomeren mit einer anionischen Gruppe, und zu 0 bis 10 Gew.-% aus weiteren vinylisch polymerisierbaren Monomeren, und
das Polymer (II) ein vom Polymer (I) verschiedenes Vinylpolymer oder ein Polysaccharid oder ein Derivat eines Polysaccharids ist, enthaltend 88 bis 100 % neutrale Monomereinheiten und bis zu 12 Gew.-% polymerisierte Monomereinheiten mit ionischen Resten,
wobei das Polymer (II) ein Copolymer aus Methylmethacrylat und Ethylacrylat, , ein Copolymer aus Methylmethacrylat, Ethylacrylat und Trimethylammoniumethylmethacrylat, Polyvinylacetat (PVAc), Hydroxyethylcellulose (HEC), Ethylcellulose (EC) oder eine Mischung der genannten Polymere ist,
zur Herstellung einer überzogenen Arzneiform, bestehend aus einem wirkstoffhaltigen Kern und einem polymeren Überzug aus der Mischung der Polymere (I) und (II), wobei der Polymermischung pharmazeutisch übliche Hilfsstoffe beigemengt sein können,
**dadurch gekennzeichnet, dass**
die Glastemperatur des Polymeren (I) nicht mehr als 70 °C beträgt und ein Wirkstofffreigabeprofil erhalten wird, bei welchem der Wirkstoff im Vergleich zu einer mit dem Polymer (I) allein überzogenen Arzneiform, beginnend beim gleichen pH-Wert jedoch verlangsamt freigesetzt wird.

2. Verwendung nach Anspruch 1, **dadurch gekennzeichnet, dass** das Polymer (I) ein Copolymer aus 10 bis 30 Gew.-%, Methylmethacrylat, 50 bis 70 Gew.-% Methylacrylat und 5 bis 15 Gew.-% Methacrylsäure ist.

3. Verwendung nach Anspruch 1, **dadurch gekennzeichnet, dass** das Polymer (I) ein Copolymer ist, welches sich aus
20 bis 34 Gew.-% Methacrylsäure und/oder Acrylsäure,
20 bis 69 Gew.-% Methylacrylat und
0 bis 40 Gew.-% Ethylacrylat und/oder gegebenenfalls
0 bis 10 Gew.-% weiteren vinylisch copolymerisierbarer Monomeren
zusammensetzt, mit der Maßgabe, dass die Glastemperatur des Copolymers höchstens 60 °C beträgt.

4. Verwendung nach Anspruch 1, **dadurch gekennzeichnet, dass** das Polymer (I) ein Copolymer ist, welches sich aus
20 bis 33 Gew.-% Methacrylsäure und/oder Acrylsäure,
5 bis 30 Gew.-% Methylacrylat und
20 bis 40 Gew.-% Ethylacrylat und
größer 10 bis 30 Gew.-% Butylmethacrylat und
gegebenenfalls
0 bis 10 Gew.-% weiteren vinylisch copolymerisierbarer Monomeren, wobei sich die Anteile der Monomeren zu 100 Gew.-% addieren,
zusammensetzt, mit der Maßgabe, daß die Glastemperatur des Copolymers 55 bis 70 °C beträgt.

5. Verwendung nach Anspruch 1, **dadurch gekennzeichnet, dass** das Polymer (II) ein Copolymer aus 20 bis 40 Gew.-% Ethylacrylat und 60 bis 80 Gew.-% Methylmethacrylat ist.

6. Verwendung nach Anspruch 1, **dadurch gekennzeichnet, dass** das Polymer (II) ein Copolymer, aus 50 - 70 Gew.-% Methylmethacrylat, 20 - 40 Gew.-% Ethylacrylat und 12 - 2 Gew.-Trimethylammoniumethylmethacrylat-Chlorid ist.

7. Verwendung nach einem oder mehreren der Ansprüche 1 bis 6, **dadurch gekennzeichnet, dass** der Polymerüberzug 2 bis 20 Gew.-% in Bezug auf das Gewicht des wirkstoffhaltigen Kerns ausmacht.

8. Verwendung nach einem oder mehreren der Ansprüche 1 bis 7, **dadurch gekennzeichnet, dass** der Wirkstoff bei dem pH-Wert, bei dem sich das Polymer (I) aufzulösen beginnt, im Freisetzungstest nach USP in 60 Minuten zu weniger als 50 % freigesetzt wird.

9. Verwendung nach einem oder mehreren der Ansprüche 1 bis 8, **dadurch gekennzeichnet, dass** die überzogene Arzneiform in Form von Pellets vorliegt, die in einer multipartikulären Arzneiform, insbesondere in pellethaltigen Tabletten, Minitabletten, Kapseln, Sachets oder Trockensäften enthalten sind.

10. Arzneiform, bestehend aus einem wirkstoffhaltigen Kern, der mit einem polymeren Mischüberzug überzogen ist, **dadurch gekennzeichnet, dass** der Mischüberzug eine Mischung aus 2 bis 60 Gew.-% eines Polymeren (I) mit 40 bis 98 Gew.-% eines Polymeren (II) ist, wobei der Polymermischung pharmazeutisch übliche Hilfsstoffe beigemengt sein können,
**dadurch gekennzeichnet, dass**
das Polymer (I) ein Copolymer aus 10 bis 30 Gew.-%, Methylmethacrylat, 50 bis 70 Gew.-% Methylacrylat und 5 bis 15 Gew.-% Methacrylsäure ist und das Polymer (II) ein vom Polymer (I) verschiedenes Vinylpolymer oder ein Polysaccharid oder ein Derivat eines Polysaccharids ist, das sich zu 88 bis 100 % aus neutralen Monomereinheiten zusammensetzt und bis zu 12 Gew.-% Monomereinheiten mit ionischen Resten enthalten kann,
wobei das Polymer (II) ein Copolymer aus Methylmethacrylat und Ethylacrylat, ein Copolymer aus Methylmethacrylat, Ethylacrylat und Trimethylammoniumethylmethacrylat, Polyvinylacetat (PVAc), Hydroxyethylcellulose (HEC), Ethylcellulose (EC) oder eine Mischung der genannten Polymere ist,
und ein Wirkstofffreigabeprofil erhalten wird, bei welchem der Wirkstoff im Vergleich zu einer mit dem Polymer (I) allein überzogenen Arzneiform, beginnend beim gleichen pH-Wert jedoch verlangsamt freigesetzt wird.

11. Arzneiform nach Anspruch 10, **dadurch gekennzeichnet, dass** das Polymer (II) ein Copolymer aus 20 bis 40 Gew.-% Ethylacrylat und 60 bis 80 Gew.-% Methylmethacrylat ist.

12. Arzneiform nach Anspruch 10, **dadurch gekennzeichnet, dass** das Polymer (II) ein Copolymer, aus 50 - 70 Gew.-% Methylmethacrylat, 20 - 40 Gew.-% Ethylacrylat und 12 - 2 Gew.-Trimethylammoniumethylmethacrylat-Chlorid ist.

13. Arzneiform nach einem oder mehreren der Ansprüche 10 bis 12, **dadurch gekennzeichnet, dass** der Polymerüberzug 2 bis 20 Gew.-% in Bezug auf das Gewicht des wirkstoffhaltigen Kerns ausmacht.

14. Arzneiform nach einem oder mehreren der Ansprüche 10 bis 13, **dadurch gekennzeichnet, dass** der Wirkstoff bei dem pH-Wert, bei dem sich das Polymer (I) aufzulösen beginnt, im Freisetzungstest nach USP in 60 Minuten zu weniger als 50 % freigesetzt wird.

15. Arzneiform nach einem oder mehreren der Ansprüche 10 bis 14, **dadurch gekennzeichnet, dass** sie in Form einer multipartikulären Arzneiform, insbesondere als pellethaltige Tablette, Minitablette, Kapsel, Sachet oder Trockensaft vorliegt.

## Claims

1. Use of a mixture of 2 to 60% by weight of one or more polymers (I) with 40 to 98% by weight of one or more polymers (II), where
polymer (I) is a (meth) acrylate copolymer comprising 90 to 100% by weight free radically polymerized units of 66 to 95% by weight of C₁- to C₄-alkyl esters of acrylic or of methacrylic acid and 5 to 34% by weight units of (meth) acrylate monomers having an anionic group, and 0 to 10% by weight of further vinylically polymerizable monomers, and
polymer (II) is a vinyl polymer different from polymer (I) or a polysaccharide or a derivative of a polysaccharide comprising 88 to 100% neutral monomer units and up to 12% by weight polymerized monomer units having ionic radicals,
wherein polymer (II) is a copolymer of methyl methacrylate and ethyl acrylate, a copolymer of methyl methacrylate, ethyl acrylate and trimethylammoniumethyl methacrylate, polyvinyl acetate (PVAc), hydroxyethylcellulose (HEC), ethylcellulose (EC) or a mixture of said polymers,
for the production of a coated pharmaceutical form consisting of an active ingredient-containing core and a polymeric coating of the mixture of polymers (I) and (II), wherein pharmaceutically usual excipients can be admixed with the polymer mixture,
**characterized in that**
the glass transition temperature of polymer (I) is not more than 70°C, and an active ingredient release profile in which the active ingredient is released by comparison with a pharmaceutical form coated with polymer (I) alone starting at the same pH but more slowly is attained.

2. Use according to Claim 1, **characterized in that** polymer (I) is a copolymer of 10 to 30% by weight methyl methacrylate, 50 to 70% by weight methyl acrylate and 5 to 15% by weight methacrylic acid.

3. Use according to Claim 1, **characterized in that** polymer (I) is a copolymer which is composed of
20 to 34% by weight methacrylic acid and/or acrylic acid,
20 to 69% by weight methyl acrylate and
0 to 40% by weight ethyl acrylate and/or optionally
0 to 10% by weight further vinylically copolymerizable monomers,
with the proviso that the glass transition temperature of the copolymer does not exceed 60°C.

4. Use according to Claim 1, **characterized in that** polymer (I) is a copolymer which is composed of
20 to 33% by weight methacrylic acid and/or acrylic acid,
5 to 30% by weight methyl acrylate and
20 to 40% by weight ethyl acrylate and
more than 10 to 30% by weight butyl methacrylate and optionally
0 to 10% by weight further vinylically copolymerizable monomers, where the proportions of the monomers add up to 100% by weight,
with the proviso that the glass transition temperature of the copolymer is 55 to 70°C.

5. Use according to Claim 1, **characterized in that** polymer (II) is a copolymer of 20 to 40% by weight ethyl acrylate and 60 to 80% by weight methyl methacrylate.

6. Use according to Claim 1, **characterized in that** polymer (II) is a copolymer of 50-70% by weight methyl methacrylate, 20-40% by weight ethyl acrylate and 12-2 by weight trimethylammoniumethyl methacrylate chloride.

7. Use according to one or more of Claims 1 to 6, **characterized in that** the polymer coating amounts to 2 to 20% by weight in relation to the weight of the active ingredient-containing core.

8. Use according to one or more of Claims 1 to 7, **characterized in that** the release of active ingredient at the pH at which polymer (I) starts to dissolve, in the USP release test, is less than 50% in 60 minutes.

9. Use according to one or more of Claims 1 to 8, **characterized in that** the coated pharmaceutical form is in the form of pellets which are present in a multiparticulate pharmaceutical form, in particular in pellet-containing tablets, minitablets, capsules, sachets or reconstitutable powders.

10. Pharmaceutical form consisting of an active ingredient-containing core which is coated with a mixed polymeric coating, **characterized in that** the mixed coating is a mixture of 2 to 60% by weight of a polymer (I) with 40 to 98% by weight of a polymer (II), wherein pharmaceutically usual excipients can be admixed with the polymer mixture,
**characterized in that**
polymer (I) is a copolymer of 10 to 30% by weight methyl methacrylate, 50 to 70% by weight methyl acrylate and 5 to 15% by weight methacrylic acid, and
polymer (II) is a vinyl polymer different from polymer (I) or a polysaccharide or a derivative of a polysaccharide which is composed to the extent of 88 to 100% of neutral monomer units and may comprise up to 12% by weight monomer units having ionic radicals,
wherein polymer (II) is a copolymer of methyl methacrylate and ethyl acrylate, a copolymer of methyl methacrylate, ethyl acrylate and trimethylammoniumethyl methacrylate, polyvinyl acetate (PVAc), hydroxyethylcellulose (HEC), ethylcellulose (EC) or a mixture of said polymers,
and an active ingredient release profile in which the active ingredient is released by comparison with a pharmaceutical form coated with polymer (I) alone starting at the same pH but more slowly is attained.

11. Pharmaceutical form according to Claim 10, **characterized in that** polymer (II) is a copolymer of 20 to 40% by weight ethyl acrylate and 60 to 80% by weight methyl methacrylate.

12. Pharmaceutical form according to Claim 10, **characterized in that** polymer (II) is a copolymer of 50-70% by weight methyl methacrylate, 20-40% by weight ethyl acrylate and 12-2 by weight trimethylammoniumethyl methacrylate chloride.

13. Pharmaceutical form according to one or more of Claims 10 to 12, **characterized in that** the polymer coating amounts to 2 to 20% by weight in relation to the weight of the active ingredient-containing core.

14. Pharmaceutical form according to one or more of Claims 10 to 13, **characterized in that** the release of active ingredient at the pH at which polymer (I) starts to dissolve, in the USP release test, is less than 50% in 60 minutes.

15. Pharmaceutical form according to one or more of Claims 10 to 14, **characterized in that** it is in the form of a multiparticulate pharmaceutical form, in particular pellet-containing tablet, minitablet, capsule, sachet or reconstitutable powder.

## Revendications

1. Utilisation d'un mélange de 2 à 60 % en poids d'un ou de plusieurs polymères (I) avec 40 à 98 % en poids d'un ou de plusieurs polymères (II),
le polymère (I) étant un copolymère de (méth)acrylate, contenant 90 à 100 % en poids de motifs, polymérisés par voie radicalaire, constitués de 66 à 95 % en poids d'esters alkyliques en C₁-C₄ de l'acide acrylique ou de l'acide méthacrylique et de 5 à 34 % en poids de motifs de monomères (méth)acrylate comportant un groupe anionique, et 0 à 10 % en poids d'autres monomères copolymérisables avec des composés vinyliques, et
le polymère (II) étant un polymère vinylique différent du polymère (I) ou un polysaccharide ou un dérivé d'un polysaccharide, contenant 88 à 100 % de motifs monomères neutres et jusqu'à 12 % en poids de motifs monomères polymérisés, comportant des radicaux ioniques,
le polymère (II) étant un copolymère de méthacrylate de méthyle et acrylate d'éthyle, un copolymère de méthacrylate de méthyle, acrylate d'éthyle et méthacrylate de triméthylammoniuméthyle, le poly(acétate de vinyle) (PVAc), l'hydroxyéthylcellulose (HEC), l'éthylcellulose (EC) ou un mélange desdits polymères,
pour la fabrication d'une forme pharmaceutique enrobée, consistant en un noyau contenant une substance active et en un enrobage polymère à base du mélange des polymères (I) et (II), des adjuvants pharmaceutiquement usuels pouvant être ajoutés au mélange de polymères,
**caractérisée en ce que**
la température de transition vitreuse du polymère (I) n'excède pas 70 °C et est obtenu un profil de libération de substance active dans lequel la substance active est libérée, en comparaison d'une forme pharmaceutique enrobée avec le polymère (I) seul, initialement au même pH mais de façon ralentie.

2. Utilisation selon la revendication 1 **caractérisée en ce que** le polymère (I) est un copolymère de 10 à 30 % en poids de méthacrylate de méthyle, 50 à 70 % en poids d'acrylate de méthyle et 5 à 15 % en poids d'acide méthacrylique.

3. Utilisation selon la revendication 1 **caractérisée en ce que** le polymère (I) est un copolymère qui se compose de
20 à 34 % en poids d'acide méthacrylique et/ou d'acide acrylique,
20 à 69 % en poids d'acrylate de méthyle et
0 à 40 % en poids d'acrylate d'éthyle et/ou éventuellement
0 à 10 % en poids d'autres monomères copolymérisables avec des composés vinyliques,
étant entendu que la température de transition vitreuse du copolymère est d'au maximum 60 °C.

4. Utilisation selon la revendication 1 **caractérisée en ce que** le polymère (I) est un copolymère qui se compose de
20 à 33 % en poids d'acide méthacrylique et/ou d'acide acrylique,
5 à 30 % en poids d'acrylate de méthyle et
20 à 40 % en poids d'acrylate d'éthyle et
plus de 10 à 30 % en poids de méthacrylate de butyle et éventuellement
0 à 10 % en poids d'autres monomères copolymérisables avec des composés vinyliques,
la somme des proportions des monomères étant égale à 100 % en poids,
étant entendu que la température de transition vitreuse du copolymère est dans la plage de 55 à 70 °C.

5. Utilisation selon la revendication 1 **caractérisée en ce que** le polymère (II) est un copolymère de 20 à 40 % en poids d'acrylate d'éthyle et 60 à 80 % en poids de méthacrylate de méthyle.

6. Utilisation selon la revendication 1 **caractérisée en ce que** le polymère (II) est un copolymère de 50 - 70 % en poids de méthacrylate de méthyle, 20 - 40 % en poids d'acrylate d'éthyle et 12 - 2 en poids de chlorure-méthacrylate de triméthylammoniuméthyle.

7. Utilisation selon une ou plusieurs des revendications 1 à 6, **caractérisé en ce que** l'enrobage polymère représente 2 à 20 % en poids par rapport au poids du noyau contenant une substance active.

8. Utilisation selon une ou plusieurs des revendications 1 à 7, **caractérisé en ce que** dans le test de libération selon USP la substance active est libérée à raison de moins de 50 % en 60 minutes, au pH auquel le polymère (I) commence à se dissoudre.

9. Utilisation selon une ou plusieurs des revendications 1 à 8, **caractérisé en ce que** la forme pharmaceutique enrobée se trouve sous forme de granules qui sont contenus dans une forme galénique multiparticulaire, en particulier en comprimés, minicomprimés, gélules, sachets ou poudres solubles, contenant des granules.

10. Forme pharmaceutique, consistant en un noyau contenant une substance active, qui est enrobé avec un enrobage mixte polymère, **caractérisée en ce que** l'enrobage mixte est un mélange de 2 à 60 % en poids d'un polymère ( I ) avec 40 à 98 % en poids d'un polymère (II), des adjuvants pharmaceutiquement usuels pouvant être ajoutés au mélange de polymères,
**caractérisée en ce que**
le polymère (I) est un copolymère de 10 à 30 % en poids de méthacrylate de méthyle, 50 à 70 % en poids d'acrylate de méthyle et 5 à 15 % en poids d'acide méthacrylique et le polymère (II) est un polymère vinylique différent du polymère (I) ou un polysaccharide ou un dérivé d'un polysaccharide, qui se compose à raison de 88 à 100 % de motifs monomères neutres et peut contenir jusqu'à 12 % en poids de motifs monomères comportant des radicaux ioniques,
le polymère (II) étant un copolymère de méthacrylate de méthyle et acrylate d'éthyle, un copolymère de méthacrylate de méthyle, acrylate d'éthyle et méthacrylate de triméthylammoniuméthyle, le poly(acétate de vinyle) (PVAc), l'hydroxyéthylcellulose (HEC), l'éthylcellulose (EC) ou un mélange desdits polymères,
et est obtenu un profil de libération de substance active dans lequel la substance active est libérée, en comparaison d'une forme pharmaceutique enrobée avec le polymère (I) seul, initialement au même pH mais de façon ralentie.

11. Forme pharmaceutique selon la revendication 10, **caractérisée en ce que** le polymère (II) est un copolymère de 20 - 40 % en poids d'acrylate d'éthyle et 60 à 80 % en poids de méthacrylate de méthyle.

12. Forme pharmaceutique selon la revendication 10, **caractérisée en ce que** le polymère (II) est un copolymère de 50 - 70 % en poids de méthacrylate de méthyle, 20 - 40 % en poids d'acrylate d'éthyle et 12 - 2 en poids de chlorure-méthacrylate de triméthylammoniuméthyle.

13. Forme pharmaceutique selon une ou plusieurs des revendications 10 à 12, **caractérisée en ce que** l'enrobage polymère représente 2 à 20 % en poids par rapport au poids du noyau contenant une substance active.

14. Forme pharmaceutique selon une ou plusieurs des revendications 10 à 13, **caractérisée en ce que** dans le test de libération selon USP la substance active est libérée à raison de moins de 50 % en 60 minutes, au pH auquel le polymère (I) commence à se dissoudre.

15. Forme pharmaceutique selon une ou plusieurs des revendications 10 à 14, **caractérisée en ce qu'**elle se trouve sous forme d'une forme galénique multiparticulaire, en particulier sous forme de comprimé, minicomprimé, gélule, sachet ou poudre soluble, contenant des granules.
